(19) Europäisches Patentamt
European Patent Office
Office européen des brevets

(11) Numéro de publication: 0 162 776
B1

(12) **FASCICULE DE BREVET EUROPÉEN**

(45) Date de publication du fascicule du brevet: 19.04.89

(21) Numéro de dépôt: 85400927.1

(22) Date de dépôt: 13.05.85

(51) Int. Cl.⁴: C 07 D 513/04, C 07 D 471/04, C 07 D 265/36, C 07 D 279/16, C 07 D 498/04, C 07 D 215/36, C 07 D 413/04, C 07 D 417/04, C 07 D 401/04, A 61 K 31/535, A 61 K 31/54

(54) **Dérivés hétérocycliques, leurs procédés de préparation, médicaments les contenant, utiles notamment comme inhibiteurs de l'aldose réductase.**

(30) Priorité: 18.05.84 FR 8407791
05.03.85 FR 8503236

(43) Date de publication de la demande: 27.11.85 Bulletin 85/48

(45) Mention de la délivrance du brevet: 19.04.89 Bulletin 89/16

(84) Etats contractants désignés: AT BE CH DE FR GB IT LI LU NL SE

(56) Documents cité:
GB-A-1 173 942
US-A-3 923 709

CHEMICAL ABSTRACTS, vol. 101, no. 8, 1984, page 616, résumé no. 55016u, Columbus, Ohio, US; D.R.SHRIDHAR et al.: "Synthesis and pharmacology of some new oxime ethers and alkanoic acid derivatives derived from 6-acetyl-2H-1,4-benzoxazin-ad-benzothiazin-3(4H)-ones"

(73) Titulaire: CARPIBEM, Société Anonyme dite :, 128, rue Danton, F-92506 Rueil Malmaison Cédex (FR)

(72) Inventeur: Teulon, Jean- Marie, 13, Avenue Guibert, F-78170 La Celle- Saint- Cloud (FR)

(74) Mandataire: Portal, Gérard, Cabinet Beau de Loménie 55, rue d'Amsterdam, F-75008 Paris (FR)

EP 0 162 776 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1989

**Description**

La présente invention concerne des dérivés hétérocycliques de formule (I). Elle concerne également les procédés de préparation desdits produits et leurs applications en thérapeutique.

On connaît des dérivés hétérocycliques par le document CHEMICAL ABSTRACTS, vol 101, N°8, 1984, résumé 55016u, présentant uniquement des propriétés anti-inflammatoires modérées et des activités sur le système nerveux central. Aucune activité inhibitrice de l'aldose réductase n'est décrite. Les dérivés hétérocycliques décrits sont différents de ceux de l'invention exposée ci-après en ce qu'ils mettent en avant la structure éther d'oxime de 6-acétyl benzoxazine ou benzothiazine.

Par ailleurs, on décrit dans le document GB-A-1 173 942, ou dans CA, vol 72, 1970, résumé 55474z, des intermédiaires de synthèse de dérivés de benzoxazine à activité anti-inflammatoire ainsi que sur le système nerveux central. De tels intermédiaires sont exclus dans les composés de formule (I) selon l'invention décrite ci-après par l'exclusion du cas où Z = Y = oxygène quand X = CH.

Egalement, on décrit dans le document CA, vol 79, 1973, résumé 87343s, des intermédiaires de synthèse d'amides dérivés de benzoxazine à activité anti-inflammatoire et antalgique. Ces intermédiaires sont exclus par l'exclusion dans les composés de formule (I) de l'invention ci-après décrite de la possibilité d'avoir Z = Y = oxygène quand X = CH.

La présente invention fournit de nouveaux composés, caractérisés en ce qu'ils répondent à la formule:

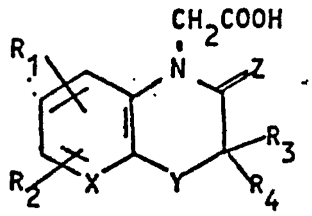

(I)

dans laquelle

| | |
|---|---|
| Z | représente l'atome de soufre, mais peut représenter également l'oxygène quand Y représente le soufre ou quand X = N et Y = NH; |
| Y | représente l'atome d'oxygène ou l'atome de soufre ou un méthylène; |
| Y | peut en outre représenter NH quand X est l'azote; |
| X | représente CH ou l'atome d'azote; |
| $R_1$ et $R_2$ | peuvent représenter l'hydrogène, un halogène, un groupement trifluorométhyle, méthoxy, thiométhyle, thiotrifluorométhyle, trifluorométhoxy; |
| $R_3$ et $R_4$ | peuvent représenter l'hydrogène, un alkyle inférieur en $C_1$-$C_5$ ramifié ou non ramifié, un noyau phényle ou pyridyle et leurs sels non toxiques d'addition. |

Selon un mode de réalisation avantageux, Z représente le soufre.

Selon un autre mode de réalisation avantageux, Y représente le soufre.

Selon un autre mode de réalisation avantageux, $R_1$ = H et $R_2$ represente le fluor.

Selon un autre mode de réalisation avantageux, $R_3$ = H et $R_4$ représente un méthyl ou un phényl.

Selon un autre mode de réalisation avantageux, Z = S, Y = S, X = CH, $R_2$ = $R_3$ = H, $R_1$ = H ou halogène, et $R_4$ = H, méthyle, phényle.

Les composés de formule (I) selon l'invention peuvent être synthéthisés par hydrolyse en milieu basique ou acide d'esters de formule (II):

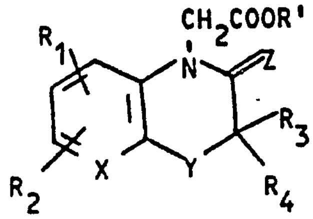

(II)

dans laquelle $R_1$, $R_2$, $R_3$, $R_4$, X, Y et Z sont définis comme ci-dessus, R' étant un groupement alkyle inférieur.

L'agent basique peut être la soude, la potasse, un carbonate de sodium ou potassium, un bicarbonate de sodium ou potassium. L'agent acide peut être un acide usuel comme l'acide chlorhydrique. La saponification sera conduite en milieu hydroalcoolique ou tétrahydrofuranne-eau ou dioxanne-eau ou un solvant miscible à l'eau. La température d'hydrolyse sera comprise entre 25 et 100°C. Pour les dérivés où Z = S, on utilisera optimalement la soude ou la potasse en milieu hydroalcoolique à 25°C ou le bicarbonate de potassium en milieu hydroalcoolique à l'ébullition.

Les composés de formule (II) dans lesquels Z = S sont synthétisés à partir des dérivés dans lesquels Z = O par action de $P_2S_5$ dans un solvant organique usuel tel que le dichlorométhane, le chloroforme, le benzène, le toluène, le xylène, la pyridine, le tétrahydrofuranne, le dioxanne, l'acétronitrile, le HMPT en présence ou non d'une base comme un bicarbonate de sodium ou potassium ou la triéthylamine à une température comprise entre 20 et 140°C, ou par action du réactif de Lawesson ou d'un réactif de thiolation analogue dans un solvant organique, tel que le benzène, le toluène, le xylène ou le diméthoxyéthane à une température comprise entre 20 et 140°C. Optimalement, on utilisera le $P_2S_5$ dans le chloroforme à une température comprise entre 20 et 61°C.

Les esters de formule II dans lesquels Z = O sont obtenus par action d'un halogénoacétate d'alkyle sur les dérivés NH de formule III préalablement métallés à l'aide d'agents courants de métallation dans des solvants classiques pour ce type de réaction, tels que le diméthylformamide, par exemple:

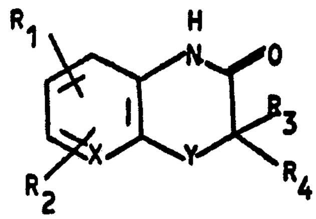

(III)

Dans la formule III, $R_1$, $R_2$, $R_3$, $R_4$, X et Y sont définis comme ci-dessus.

Les dérivés de formule III sont obtenus par cyclisation des aminoesters ou des aminoacides de formule IV:

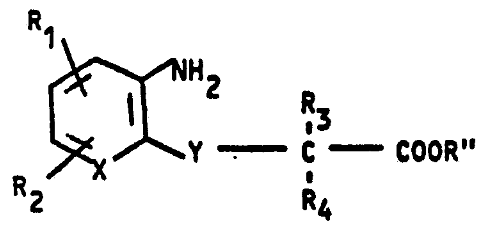

(IV)

dans laquelle $R_1$, $R_2$, $R_3$, $R_4$, X et Y sont définis comme ci-dessus, R" étant l'hydrogène ou un alkyle inférieur.

Les dérivés de formule III, dans lesquels Y = S, peuvent également être synthétisés directement par action d'un dérivé de formule V sur un halogénoacétate d'alkyle ou l'acide ou son chlorure d'acide de formule VI.

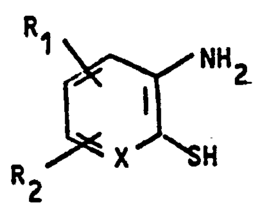

(V)

$$W - \overset{R_3}{\underset{R_4}{\overset{|}{\underset{|}{C}}}} - CO\,U \qquad (VI)$$

Dans la formule V, $R_1$, $R_2$ et X sont définis comme ci-dessus; dans la formule VI, $R_3$ et $R_4$ sont définis comme ci-dessus, W représente un halogène, U représente OH, OR" ou le chlore, R" étant défini comme ci-dessus.

Les dérivés de formule III dans lesquels Y = $CH_2$ et X = CH peuvent également être synthétisés par cyclisation à l'acide, de méthode connue en soi, par exemple le chlorure d'aluminium, de dérivés de formule VII:

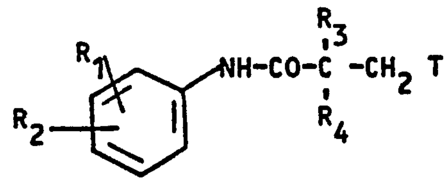

(VII)

Dans la formule VII, $R_1$, $R_2$, $R_3$ et $R_4$ sont définis comme ci-dessus, T représente l'atome de chlore ou de brome.

Les composés de formule IV sont synthétisés par hydrogénation des dérivés nitrés de formule VIII:

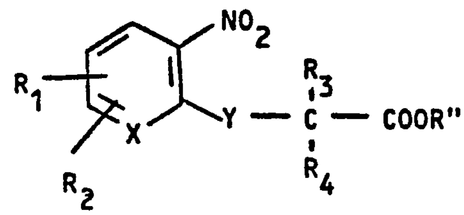

(VIII)

dans laquelle $R_1$, $R_2$, $R_3$, $R_4$, X, Y et R'' sont définis comme ci-dessus.

Les dérivés de formule VIII sont obtenus par action, dans le cas où Y = N, d'un ester de la glycine ou, dans le cas où Y = S, d'un ester d'un acide mercaptoacétique ou de l'acide lui-même sur les dérivés nitrés de formule IX:

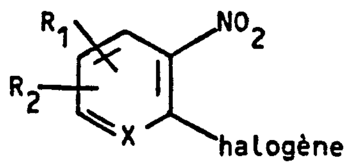

(IX)

dans laquelle $R_1$, $R_2$ et X sont définis comme ci-dessus et, dans le cas où Y = O pour X = CH ou Y = S, peuvent également être obtenus par action d'un halogénoacétate d'alkyle convenablement substitué sur un dérivé de formule X préalablement métallé si necessaire.

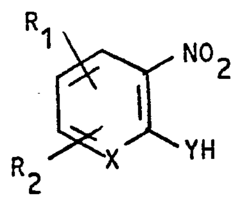

(X)

Dans la formule X, $R_1$, $R_2$, X et Y sont définis comme ci-dessus.

Selon l'invention, on propose des compositions thérapeutiques utiles notamment pour le traitement des troubles périphériques consécutifs au diabète (cataracte, neuropathie), caractérisées en ce qu'elles renferment, en association avec un excipient physiologiquement acceptable, au moins un composé de formule I ou un de ses sels non toxiques d'addition.

Pour tous les dérivés de l'invention, on pourra réaliser la salification de la fonction acide par un métal ou un alcalino-terreux, tel que sodium ou potassium, une base telle qu'une amine comme la dicyclohexylamine, un aminoalcool comme l'aminoéthanol ou un aminoacide comme la lysine.

Lorsque X = Y = N, la salification pourra se faire par exemple avec un acide tel que HCl, HBr ou un acide organique habituellement utilisé.

D'autres caractéristiques et avantages de l'invention seront mieux compris à la lecture qui va suivre de quelques exemples de préparation de composés de formule (I) précitée.

Le tableau II ci-après donne la forme développée de certains produits.

**Exemple 1**

3-nitro 2-mercaptoacétate d'éthyle-pyridine

Formule VIII: $R_1 = R_2 = R_3 = R_4 = H$; $X = N$; $Y = S$; $R'' = C_2H_5$

On porte au reflux durant 7 h une solution de 50 g de 3-nitro 2-chloropyridine, 38 g de mercaptoacétate d'éthyle dans 400 ml d'éthanol contenant 30 g de bicarbonate de sodium. La solution est ensuite concentrée sous vide puis, après refroidissement, le résidu additionné d'eau et de glace est extrait à l'éther. La phase éthérée, lavée à l'eau, est séchée et, le solvant évaporé, on récupère un résidu qui cristallise dans l'éther isopropylique. Les cristaux sont essorés et séchés et on obtient 49 g de 3-nitro 2-mercaptoacétate d'éthyle-pyridine sous forme de cristaux de point de fusion 58° C.

**Exemple 2**

6-chloro 3-nitro 2-mercaptoacétate d'éthyle-pyridine

Formule VIII: $R_1$ = 6-chloro; $R_2 = R_3 = R_4 = H$; $X = N$; $Y = S$; $R'' = C_2H_5$

Selon le mode opératoire de l'exemple 1, mais en utilisant 25 g de 2,6-dichloro 3-nitropyridine à 90 % et 14 g de mercaptoacétate d'éthyle, on obtient 32,5 g de 6-chloro 3-nitro 2-mercaptoacétate d'éthyle-pyridine sous forme d'une huile utilisée brute pour l'étape ultérieure.

**Exemple 3**

3-nitro 2-mercapto α-méthylacétique acide-pyridine

Formule VIII: $R_1 = R_2 = R_3 = H$; $R_4 = CH_3$; $X = N$; $Y = S$; $R'' = H$

Selon le mode opératoire de l'exemple 1, mais en utilisant 46 g de 3-nitro 2-chloropyridine et 32 g de 2-mercaptopropionique acide, on obtient, après acidification à l'acide acétique, 50 g de 3-nitro 2-mercapto α-méthylacétique acide-pyridine sous forme de cristaux de point de fusion 135° C.

**Exemple 4**

3-nitro 2-aminoacétate d'éthyle-pyridine

Formule VIII: $R_1 = R_2 = R_3 = R_4 = H$; $X = Y = N$; $R'' = C_2H_5$

On porte au reflux durant 3 h une solution de 20 g de 3-nitro 2-chloropyridine et de 32 g de glycinate d'éthyle dans 100 ml d'éthanol. La solution est ensuite concentrée sous vide et, après refroidissement, puis addition d'eau et de glace, on extrait à l'éther. La phase éthérée est lavée à l'eau puis séchée et l'éther est évaporé sous vide. On récupère 28 g de 3-nitro 2-aminoacétate d'éthyle-pyridine sous forme d'une huile utilisée brute pour l'étape suivante.

**Exemple 5**

4-trifluorométhyl 2-nitrophénylthioacétate d'éthyle

Formule VIII: $R_1 = 4\text{-}CF_3$; $R_2 = R_3 = R_4 = H$; $X = CH$; $Y = S$; $R'' = C_2H_5$

Selon le mode opératoire de l'exemple 1, mais en utilisant 38 g de 4-trifluorométhyl 2-nitrochlorobenzène et 21,5 g de mercaptoacétate d'éthyle, on obtient, après distillation du résidu [E. (2 mmHg) = 155 - 160° C], 29 g de 4-trifluorométhyl 2-nitrophénylthioacétate d'éthyle sous forme de cristaux de point de fusion 42 - 45° C.

**Exemple 6**

5-fluoro 2-nitrophénylthioacétate d'éthyle

Formule VIII: $R_1 = 5\text{-F}$; $R_2 = R_3 = R_4 = H$; $X = CH$; $Y = S$; $R = C_2H_5$

Selon le mode opératoire de l'exemple 1, mais en utilisant 83 g de 2,4-difluoronitrobenzène et 63 g de mercaptoacétate d'éthyle, on obtient après distillation du résidu [E. (1mmHg) = 155 - 160° C] 85 g de 5-fluoro 2-nitrophénylthioacétate d'éthyle sous forme de cristaux de point de fusion 58° C.

**Exemple 7**

4-fluoro 2-nitrophénylthioacétate d'éthyle

Formule VIII: $R_1 = 4\text{-F}$; $R_2 = R_3 = R_4 = H$; $X = CH$; $Y = S$; $R'' = C_2H_5$

Selon le mode opératoire de l'exemple 1, mais à partir de 73 g de 2,5-difluoronitrobenzène et de 56 g de mercaptoacétate d'éthyle, on obtient après cristallisation du résidu dans le pentane 75 g de 4-fluoro 2-nitrophénylthioacétate d'éthyle sous forme de cristaux de point de fusion < 50° C.

**Exemple 8**

6-chloro 2-nitrophénylthioacétate d'éthyle

Formule VIII: $R_1 = 6\text{-Cl}$; $R_2 = R_3 = R_4 = H$; $X = CH$; $Y = S$; $R'' = C_2H_5$

Selon le mode opératoire de l'exemple 1, mais en utilisant 100 g de 2,3-dichloronitrobenzène et 63 g de mercaptoacétate d'éthyle, on obtient après filtration sur gel de silice 22,5 g de 6-chloro 2-nitrophénylthioacétate d'éthyle sous forme d'une huile utilisée brute pour l'étape suivante.

**Exemple 9**

4-méthoxy 2-nitrophénylthioacétate d'éthyle

Formule VIII: $R_1 = 4\text{-OCH}_3$; $R_2 = R_3 = R_4 = H$; $X = CH$; $Y = S$; $R'' = C_2H_5$

Selon le mode opératoire de l'exemple 1, mais à partir de 100 g de 2-chloro 5-méthoxynitrobenzène et de 68 g de mercaptoacétate d'éthyle, on obtient après cristallisation du résidu dans l'éther isopropylique 55 g de 4-méthoxy 2-nitrophénylthioacétate d'éthyle sous forme de cristaux de point de fusion 75° C.

**Exemple 10**

4-trifluorométhyl 5-chloro 2-nitrophénylthioacétate d'éthyle

Formule VIII: $R_1 = 4\text{-CF}_3$; $R_2 = 5\text{-Cl}$; $R_3 = R_4 = H$; $X = CH$; $Y = S$; $R'' = C_2H_5$

Selon le mode opératoire de l'exemple 1, mais à partir de 50 g de 2,4-dichloro 5-trifluorométhylnitrobenzène et de 23 g de mercaptoacétate d'éthyle, on obtient 56 g de 4-trifluorométhyl 5-chloro 2-nitrophénylthioacétate d'éthyle sous forme de cristaux de point de fusion 85° C.

**Exemple 11**

5-chloro 2-nitrophénylthioacétate d'éthyle

Formule VIII: $R_1 = 5\text{-Cl}$; $R_2 = R_3 = R_4 = H$; $X = CH$; $Y = S$; $R'' = C_2H_5$

Selon le mode opératoire de l'exemple 1, mais en utilisant 68,8 g de 2,4-dichloronitrobenzène et 43 g de mercaptoacétate d'éthyle, on obtient après filtration sur gel de silice 45 g de 5-chloro 2-nitrophénylthioacétate d'éthyle sous forme de cristaux de point de fusion 48 - 50° C.

**Exemple 12**

4-chloro 2-nitrophénylthioacétate d'éthyle

Formule VIII: $R_1$ = 4-Cl; $R_2$ = $R_3$ = $R_4$ = H; X = CH; Y = S; R" = $C_2H_5$

Selon le mode opératoire de l'exemple 1, mais en utilisant 100 g de 2,5-dichloronitrobenzène et 63 g de mercaptoacétate d'éthyle, on obtient après cristallisation du résidu dans un mélange pentane-éther isopropylique 52 g de 4-chloro 2-nitrophénylthioacétate sous forme de cristaux de point de fusion 52° C.

**Exemple 13**

4-fluoro 2-nitrophénylthio α-méthylacétique acide

Formule VIII: $R_1$ = 4-F; $R_3$ = $CH_3$; $R_2$ = $R_4$ = H; X = CH; Y = S; R" = H

Selon le mode opératoire de l'exemple 1, mais en utilisant 15,9 g de 2,5-difluoronitrobenzène et 11 g de 2-mercaptopropionique acide, on obtient, après acidification à l'acide acétique, 21 g de 4-fluoro 2-nitrophénylthio α-méthylacétique acide sous forme de cristaux de point de fusion 116° C.

**Exemple 14**

4-chloro 2-nitrophénylthio α-méthylacétique acide

Formule VIII: $R_1$ = 4-Cl; $R_3$ = $CH_3$; $R_2$ = $R_4$ = H; X = CH; Y = S; R" = H

Selon le mode opératoire de l'exemple 1, mais en utilisant 38,4 g de 2,5-dichloronitrobenzène et 22 g de 2-mercaptopropionique acide, on obtient, après acidification à l'acide acétique, 32 g de 4-chloro 2-nitrophényl α-méthylacétique acide sous forme de cristaux de point de fusion 114° C.

**Exemple 15**

4-chloro 2-nitrophénylthio α-phénylacétate d'éthyle

Formule VIII: $R_1$ = 4-Cl; $R_3$ = phényl; $R_2$ = $R_4$ = H; X = CH; Y = S; R" = $C_2H_5$

A une solution de 10,5 g de 2-nitro 4-chlorothiophénol dans 100 ml d'éthanol contenant 2,2 g de soude dissoute dans 10 ml d'eau, on ajoute goutte à goutte sous agitation une solution de 14 g d'α-bromophénylacétate d'éthyle dans 10 ml d'éthanol. Après la fin de l'addition, l'agitation est continuée 3 h à température ambiante puis le précipité formé est essoré, lavé par un peu d'éthanol, avec une solution aqueuse de soude 5 %, puis à l'eau et séché. On récupère ainsi 11,5 g de 4-chloro 2-nitrophénylthio α-phénylacétate d'éthyle sous forme de cristaux de point de fusion 112° C.

**Exemple 16**

5-chloro 2-nitrophénylthio α-méthylacétique acide

Formule VIII: $R_1$ = 5-Cl; $R_3$ = $CH_3$; $R_2$ = $R_4$ = H; X = CH: Y = S; R" = H

Selon le mode opératoire de l'exemple 1, mais en utilisant 100 g de 2,4-dichloronitrobenzène et 55 g de 2-mercaptopropionique acide, on obtient, après acidification à l'acide acétique et cristallisation du résidu dans un mélange éther isopropylique et pentane, 76 g de 5-chloro 2-nitrophénylthio α-méthylacétique acide sous forme de cristaux de point de fusion 125° C.

**Exemple 17**

4,5-dichloro 2-nitrophénylthioacétate d'éthyle

Formule VIII: $R_1$ = 4-Cl; $R_2$ = 5-Cl; $R_3$ = $R_4$ = H; X =CH; Y = S; R" = $C_2H_5$

Selon le mode opératoire de l'exemple 1, mais en utilisant 93,2 g de 2,4,5-trichloronitrobenzène et 42 g de mercaptoacétate d'éthyle, on obtient après filtration sur gel de silice 68 g d'un mélange de 4,5-dichloro 2-nitrophénylthioacétate d'éthyle et de 2,5-dichloro 4-nitrophénylthioacétate d'éthyle qui est utilisé brut pour l'étape suivante.

**Exemple 18**

4-fluoro 2-nitrophénoxyacétate d'éthyle

Formule VIII: $R_1$ = 4-F; $R_2$ = $R_3$ = $R_4$ = H; X = CH; Y = O; R" = $C_2H_5$

A une solution de 54,7 g de 4-fluoro 2-nitrophenol dans 400 ml d'éthanol, on ajoute goutte à goutte 14 g de soude en solution dans 30 ml d'eau. Le mélange réactionnel est ensuite refroidi par un bain de glace et on additionne goutte à goutte 40 ml de chloroacétate d'éthyle sous agitation. On laisse ensuite revenir à température ambiante, puis on agite durant 4 h et on porte ensuite au reflux toujours sous agitation quatre nouvelles heures.

Le mélange réactionnel est ensuite concentré sous vide, le résidu repris par l'eau et la glace est extrait à l'éther qu'on lave à l'eau, à la soude diluée puis encore à l'eau et qu'on sèche. L'éther évaporé, on récupère 46 g de 4-fluoro 2-nitrophénoxyacétate d'éthyle sous forme d'une huile utilisée brute à l'étape suivante.

**Exemple 19**

4-fluoro 2-nitrophénylthio α-méthylacétate d'éthyle

Formule VIII: $R_1$ = 4-F; $R_3$ = $CH_3$; $R_2$ = $R_4$ = H; X = CH; Y = S; R" = $C_2H_5$

On porte au reflux durant 5 h une solution de 44,3 g de 4-fluoro 2-nitrophénylthio α-méthylacétique acide préparé à l'exemple 13 dans 300 ml d'éthanol contenant 10 ml d'acide sulfurique concentré. Le mélange réactionnel est ensuite concentré sous vide, le résidu repris à l'eau, extrait à l'éther qu'on lave avec une solution d'ammoniaque à 5 % puis essore à l'eau et qu'on sèche. Après évaporation de l'éther, on récupère 46,5 g de 4-fluoro 2-nitrophénylthio α-méthylacétate d'éthyle sous forme d'une huile qui est utilisée brute pour l'étape suivante.

**Exemple 20**

4-chloro 2-nitrophénylthio α-méthylacétate d'éthyle

Formule VIII: $R_1$ = 4-Cl; $R_3$ = $CH_3$; $R_2$ = $R_4$ = H; X = CH; Y = S; R" = $C_2H_5$

Selon le mode opératoire de l'exemple 19, mais à partir de 32 g de 4-chloro 2-nitrophénylthio α-méthylacétique acide préparé à l'exemple 14, on obtient 34 g de 4-chloro 2-nitrophénylthio α-méthylacétate d'éthyle sous forme d'une huile utilisée brute pour l'étape suivante.

**Exemple 21**

5-chloro 2-nitrophénylthio α-méthylacétate d'éthyle

Formule VIII: $R_1$ = 5-Cl; $R_3$ = $CH_3$; $R_2$ = $R_4$ = H; X = CH; Y = S; R" = $C_2H_5$

Selon le mode opératoire de l'exemple 19, mais à partir de 76 g de 5-chloro 2-nitrophénylthio α-méthylacétique acide préparé à l'exemple 16, on obtient 72 g de 5-chloro 2-nitrophénylthio α-méthylacétate d'éthyle sous forme d'une huile utilisée brute pour l'étape suivante.

**Exemple 22**

1H-pyrido[2,3-b][1,4]thiazine-2 (3H)-one

Formule III: $R_1$ = $R_2$ = $R_3$ = $R_4$ = H; X = N; Y = S

On hydrogène sous pression atmosphérique une solution de 49 g de 3-nitro 2-mercaptoacétate d'éthyle-pyridine préparés à l'exemple 1, en présence de nickel de Raney dans 500 ml de méthanol. Après l'absorption de la quantité théorique d'hydrogène, le catalyseur est séparé par filtration et la solution concentrée sous vide, puis le résidu repris au xylène est chauffé 16 h au reflux. Après refroidissement, puis addition d'hexane, les cristaux formés sont essorés puis séchés. On récupère 24 g de 1H-pyrido[2,3-b][1,4]thiazine-2 (3H)-one sous forme de cristaux de point de fusion 207° C.

**Exemple 23**

6-chloro 1H-pyrido[2,3-b][1,4]thiazine-2 (3H)-one

Formule III: $R_1$ = 6-chloro; $R_2$ = $R_3$ = $R_4$ = H; X = N; Y = S

Selon le mode opératoire de l'exemple 22, mais à partir de 14 g de 6-chloro 3-nitro 2-mercaptoacétate d'éthyle-pyridine préparés à l'exemple 2, on récupère 4,1 g de 6-chloro 1H-pyrido-[2,3-b][1,4]thiazine-2 (3H)-one sous forme de cristaux de point de fusion 240 - 245° C.

**Exemple 24**

3-méthyl 1H-pyrido[2,3-b][1,4]thiazine-2 (3H)-one

Formule III: $R_1$ = $R_2$ = $R_3$ = H; $R_4$ = $CH_3$; X = N; Y = S

Selon le mode opératoire de l'exemple 22, mais à partir de 30 g de 3-nitro 2-mercapto α-méthylacétique acide-pyridine préparés à l'exemple 3, on récupère 16 g de 3-méthyl 1H-pyrido[2,3-b]-[1,4]thiazine-2 (3H)-one sous forme de cristaux de point de fusion 178° C.

**Exemple 25**

1H-pyrido[2,3-b][1,4]pyrazine-2 (3H)-one

Formule III: $R_1$ = $R_2$ = $R_3$ = $R_4$ = H; X = Y = N

Selon le mode opératoire de l'exemple 22, mais à partir de 33 g de 3-nitro 2-aminoacétate d'éthyle-pyridine, préparés à l'exemple 4, on obtient 19 g de 1H-pyrido[2,3-b][1,4]pyrazine-2 (3H)-one sous forme de cristaux de point de fusion 278 - 282° C.

**Exemple 26**

6-trifluorométhyl 2H-1,4-benzothiazine-3 (4H)-one

Formule III: $R_1$ = 6-$CF_3$; $R_2$ = $R_3$ = $R_4$ = H; X = CH; Y = S

Selon le mode opératoire de l'exemple 22, mais à partir de 29 g de 4-trifluorométhyl 2-nitrophénylacétate d'éthyle préparés à l'exemple 5, on récupère 15 g de 6-trifluorométhyl 2H-1,4-benzothiazine-3 (4H)-one sous forme de cristaux de point de fusion 190° C.

**Exemple 27**

7-fluoro 2H-1,4-benzothiazine-3 (4H)-one

Formule III: $R_1$ = 7-F; $R_2$ = $R_3$ = $R_4$ = H; X = CH; Y = S

Selon le mode opératoire de l'exemple 22, mais à partir de 90 g de 5-fluoro 2-nitrophénylthioacétate d'éthyle, préparés à l'exemple 6, on obtient 45 g de 7-fluoro 2H-1,4-benzothiazine-3 (4H)-one sous forme de cristaux de point de fusion 215° C.

**Exemple 28**

6-fluoro 2H-1,4-benzothiazine-3 (4H)-one

Formule III: $R_1$ = 6-F; $R_2$ = $R_3$ = $R_4$ = H; X = CH; Y = S

On hydrogène sous pression atmosphérique une solution de 75 g de 4-fluoro 2-nitrophénylthioacétate d'éthyle, préparés à l'exemple 7, en présence de nickel de Raney dans 1 litre de méthanol. Après l'absorption de la quantité théorique d'hydrogène, le catalyseur est séparé par filtration et la solution est concentrée sous vide puis le résidu repris par 250 ml d'eau à 50° C et 60 ml d'acide chlorhydrique concentré est agité durant 30 min. Les cristaux formés sont essorés, lavés à l'eau puis au pentane et séchés. On récupère ainsi 46 g de 6-fluoro 2H-1,4-benzothiazine-3 (4H)-one sous forme de cristaux de point de fusion 189° C.

**Exemple 29**

8-chloro 2H-1,4-benzothiazine-3 (4H)-one

Formule III: $R_1$ = 8-Cl, $R_2$ = $R_3$ = $R_4$ = H; X = CH; Y = S

Selon le mode opératoire de l'exemple 28, mais en utilisant 22,5 g de 6-chloro 2-nitrophénylthioacétate d'éthyle, préparés à l'exemple 8, on obtient 12 g de 8-chloro 2H-1,4-benzothiazine-3 (4H)-one sous forme de cristaux de point de fusion 222° C.

**Exemple 30**

7-chloro 2H-1,4-benzothiazine-3 (4H)-one

Formule III: $R_1$ = 7-Cl; $R_2$ = $R_3$ = $R_4$ = H; X = CH; Y = S

Selon le mode opératoire de l'exemple 28, mais en utilisant 42 g de 5-chloro 2-nitrophénylthioacétate d'éthyle, préparés à l'exemple 11, on obtient 23 g de 7-chloro 2H-1,4-benzothiazine-3 (4H)-one sous forme de cristaux de point de fusion 210° C.

**Exemple 31**

6-méthoxy 2H-1,4-benzothiazine-3 (4H)-one

Formule III: $R_1 = 6\text{-}OCH_3$; $R_2 = R_3 = R_4 = H$; $X = CH$; $Y = S$

Selon le mode opératoire de l'exemple 28, mais en utilisant 45 g de 4-méthoxy 2-nitrophénylthioacétate d'éthyle, préparés à l'exemple 9, on obtient 27 g de 6-méthoxy 2H-1,4-benzothiazine-3 (4H)-one sous forme de cristaux de point de fusion 180 - 182° C.

**Exemple 32**

6-trifluorométhyl 7-chloro 2H-1,4-benzothiazine-3 (4H)-one

Formule III: $R_1 = 6\text{-}CF_3$; $R_2 = 7\text{-}Cl$; $R_3 = R_4 = H$; $X = CH$; $Y = S$

Selon le mode opératoire de l'exemple 28, mais en utilisant 56 g de 4-trifluorométhyl 5-chloro 2-nitrophénylthioacétate d'éthyle, préparés à l'exemple 10, on obtient après recristallisation dans le méthanol 30 g de 6-trifluorométhyl 7-chloro 2H-1,4-benzothiazine-3 (4H)-one sous forme de cristaux de point de fusion 234 - 235° C.

**Exemple 33**

2H-1,4-benzothiazine-3 (4H)-one

Formule III: $R_1 = R_2 = R_3 = R_4 = H$; $X = CH$; $Y = S$

On prépare une solution d'éthylate de sodium en dissolvant 4,6 g de sodium dans 200 ml d'éthanol absolu. A cette solution, on ajoute goutte à goutte 25 g d'orthoaminothiophénol puis, à la fin de l'addition, on agite durant 10 min, on refroidit par un bain de glace puis on ajoute goutte à goutte 24 ml de bromoacétate d'éthyle. Après la fin de l'addition, on agite durant 3 h à température ambiante puis on filtre le bromure de sodium formé et le filtrat est concentré sous vide. Le résidu obtenu cristallise dans l'éther. Les cristaux essorés et séchés, on obtient 23 g de 2H-1,4-benzothiazine-3 (4H)-one sous forme de cristaux de point de fusion 177° C.

**Exemple 34**

2-méthyl 2H-1,4-benzothiazine-3 (4H)-one

Formule III: $R_1 = R_2 = R_3 = H$; $R_4 = CH_3$; $X = CH$; $Y = S$

A une solution de 37,5 g d'orthoaminothiophénol dans 100 ml d'éthanol, on additionne une solution de 24 g de soude dissoute dans le minimum d'eau, on laisse sous agitation 10 min, on refroidit par un bain de glace puis on ajoute goutte à goutte 45,9 g d'α-bromopropionique acide en solution dans 50 ml d'éthanol. On laisse le mélange réactionnel revenir à température ambiante puis on porte au reflux durant 4 h. Après concentration sous vide, on additionne de l'eau et de l'acide chlorhydrique jusqu'à pH acide et les cristaux formés sont essorés, lavés à l'eau puis séchés. On récupère ainsi 41,7 g de 2-méthyl 2H-1,4-benzothiazine-3 (4H)-one sous forme de cristaux de point de fusion 126 - 127° C.

**Exemple 35**

2-phényl 2H-1,4-benzothiazine-3 (4H)-one

Formule III: $R_1 = R_2 = R_3 = H$; $R_4 =$ phényle; $X = CH$; $Y = S$

Selon le mode opératoire de l'exemple 34, mais en utilisant 12,5 g d'orthoaminothiophénol et 17 g d'α-chlorophénylacétique acide, on obtient 9,7 g de 2-phényl 2H-1,4-benzothiazine-3 (4H)-one sous forme de cristaux de point de fusion 194° C.

**Exemple 36**

6-chloro 2H-1,4-benzothiazine-3 (4H)-one

Formule III: $R_1$ = 6-Cl; $R_2$ = $R_3$ = $R_4$ = H; X = CH; Y = S

Selon le mode opératoire de l'exemple 28, mais en utilisant 52 g de 4-chloro 2-nitrophénylthioacétate d'éthyle, préparés à l'exemple 12, on obtient 27,3 g de 6-chloro 2H-1,4-benzothiazine-3 (4H)-one sous forme de cristaux de point de fusion 208°C.

**Exemple 37**

6-fluoro 2-méthyl 2H-1,4-benzothiazine-3 (4H)-one

Formule III: $R_1$ = 6-F; $R_3$ = $CH_3$; $R_2$ = $R_4$ = H; X = CH; Y = S

Selon le mode opératoire de l'exemple 28, mais en utilisant 55 g de 4-fluoro 2-nitrophénylthio α-méthylacétate d'éthyle, préparés à l'exemple 19, on obtient, après recristallisation dans l'isopropanol, 31 g de 6-fluoro 2-méthyl 2H-1,4-benzothiazine-3 (4H)-one sous forme de cristaux de point de fusion 164°C.

**Exemple 38**

6-chloro 2-méthyl 2H-1,4-benzothiazine-3 (4H)-one

Formule III: $R_1$ = 6-Cl; $R_3$ = $CH_3$; $R_2$ = $R_4$ = H; X = CH; Y = S

Selon le mode opératoire de l'exemple 28, mais en utilisant 34 g de 4-chloro 2-nitrophénylthio α-méthylacétate d'éthyle, préparés à l'exemple 20, on obtient 19 g de 6-chloro 2-méthyl 2H-1,4-benzothiazine-3 (4H)-one sous forme de cristaux de point de fusion 188°C.

**Exemple 39**

6-chloro 2-phényl 2H-1,4-benzothiazine-3 (4H)-one

Formule III: $R_1$ = 6-Cl; $R_3$ = phényle; $R_2$ = $R_4$ = H; X = CH; Y = S

Selon le mode opératoire de l'exemple 28, mais en utilisant 11,5 g de 4-chloro 2-nitrophénylthio α-phénylacétate d'éthyle, préparés à l'exemple 15, on obtient 4 g de 6-chloro 2-phényl 2H-1,4-benzothiazine-3 (4H)-one sous forme de cristaux de point de fusion 216°C.

**Exemple 40**

2,2-diméthyl 2H-1,4-benzothiazine-3 (4H)-one

Formule III: $R_1$ = $R_2$ = H; $R_3$ = $R_4$ = $CH_3$; X = CH; Y = S

Selon le mode opératoire de l'exemple 34, mais à partir de 90 g d'orthoaminothiophénol, 28,8 g de soude et 140,4 g d'α-bromoisobutyrate d'éthyle, on obtient 112 g de 2,2-diméthyl 2H-1,4-benzothiazine-3 (4H)-one sous forme de cristaux de point de fusion 155°C.

**Exemple 41**

2-parachlorophényl 2H-1,4-benzothiazine-3 (4H)-one

Formule III: $R_1$ = $R_2$ = $R_3$ = H; $R_4$ = parachlorophényl; X = CH; Y = S

Selon le mode opératoire de l'exemple 34, mais à partir de 25 g d'orthoaminothiophénol, 8 g de soude et 55 g d'α-bromoparachlorophénylacétate d'éthyle, on obtient 37,8 g de 2-parachlorophényl 2H-1,4-benzothiazine-3 (4H)-one sous forme de cristaux de point de fusion 198°C.

**Exemple 42**

2-orthochlorophényl 2H-1,4-benzothiazine-3 (4H)-one

Formule III: $R_1 = R_2 = R_3 = H$; $R_4$ = orthochlorophényl; X = CH; Y = S

Selon le mode opératoire de l'exemple 34, mais à partir de 25 g d'orthoaminothiophénol, 8 g de soude et 55 g d'α-bromoorthochlorophénylacétate d'éthyle, on obtient 37 g de 2-orthochlorophényl 2H-1,4-benzothiazine-3 (4H)-one sous forme de cristaux de point de fusion 200°C.

**Exemple 43**

6,7-dichloro 2H-1,4-benzothiazine-3 (4H)-one

Formule III: $R_1$ = 6-Cl; $R_2$ = 7-Cl; $R_3 = R_4 = H$; X = CH; Y = S

Selon le mode opératoire de l'exemple 28, mais à partir du mélange de 68 g de 4,5-dichloro 2-nitrophénylthioacétate d'éthyle et de 2,5-dichloro 4-nitrophénylthioacétate d'éthyle, préparés à l'exemple 17, on obtient 4,5 g de 6,7-dichloro 2H-1,4-benzothiazine-3 (4H)-one sous forme de cristaux de point de fusion 251 - 253°C.

**Exemple 44**

2-méthyl 2-(2'-pyridyl) 2H-1,4-benzothiazine-3 (4H)-one

Formule III: $R_1 = R_2 = H$; $R_3 = CH_3$; $R_4$ = 2'-pyridyl; X = CH; Y = S

On prépare une solution d'éthylate de sodium en dissolvant 4 g de sodium dans 300 ml d'éthanol absolu. A cette solution, on ajoute goutte à goutte 21,5 g d'orthoaminothiophénol puis, après la fin de l'addition, on agite durant 10 min. On refroidit par un bain de glace, puis on ajoute goutte à goutte 40 g d'α-bromo α-méthyl 2-pyridylacétate d'éthyle; après la fin de l'addition, on laisse revenir à température ambiante puis on continue l'agitation durant 4 h et on laisse au repos une nuit. Le précipité formé est essoré, lavé à l'eau puis à l'éthanol et séché. On récupère ainsi 25,3 g de 2-méthyl 2-(2'-pyridyl) 2H-1,4-benzothiazine-3 (4H)-one sous forme de cristaux de point de fusion 217 - 219°C.

**Exemple 45**

6-fluoro 2H-1,4-benzoxazine-3 (4H)one

Formule III: $R_1$ = 6-F; $R_2 = R_3 = R_4 = H$; X = CH; Y = O

On hydrogène sous pression atmosphérique une solution de 46 g de 4-fluoro 2-nitrophénoxyacétate d'éthyle, préparés à l'exemple 18, en présence de nickel de Raney dans 500 ml de méthanol. Après l'absorption de la quantité théorique d'hydrogène, le catalyseur est séparé par filtration et la solution est concentrée sous vide, et le résidu repris à l'éther cristallise. Les cristaux formés sont essorés, lavés à l'éther et séchés. On obtient ainsi 28 g de 6-fluoro 2H-1,4-benzoxazine-3 (4H)-one sous forme de cristaux de point de fusion 206 - 207°C.

**Exemple 46**

6-fluoro 3,4-dihydrocarbostyril

Formule III: $R_1$ = 6-F; $R_2$ = $R_3$ = $R_4$ = H; X = CH; Y = $CH_2$

A un mélange de 158 g de chlorure d'aluminium et 40 g de chlorure de sodium, on ajoute entre 180 et 220°C 31 g du N-(parafluorophényl) β-chloropropionamide puis on agite le mélange réactionnel à cette température durant 30 min. Le mélange réactionnel est ensuite détruit sur de la glace et les cristaux formés sont essorés, lavés à l'eau et séchés. On récupère ainsi après recristallisation dans l'isopropanol 14 g de 6-fluoro 3,4-dihydrocarbostyril sous forme de cristaux de point de fusion 180 - 181°C.

**Exemple 47**

6-chloro 3,4-dihydrocarbostyril

Formule III: $R_1$ = 6-Cl; $R_2$ = $R_3$ = $R_4$ = H; X = CH; Y = $CH_2$

Selon le mode opératoire de l'exemple 46, mais à partir de 142,7 g de N-(parachlorophényl) β-chloropropionamide, on récupère 66,4 g de 6-chloro 3,4-dihydrocarbostyril sous forme de cristaux de point de fusion 163 - 165°C après recristallisation dans l'acétonitrile.

**Exemple 48**

7-fluoro 3,4-dihydrocarbostyril

Formule III: $R_1$ = 7-F; $R_2$ = $R_3$ = $R_4$ = H; X = CH; Y = $CH_2$

Selon le mode opératoire de l'exemple 46, mais à partir de 90,4 g de N-(méta-fluorophényl) β-chloropropionamide, on obtient 35,8 g de 7-fluoro 3,4-dihydrocarbostyril sous forme de cristaux de point de fusion 187 - 188°C après recristallisation dans l'isopropanol.

**Exemple 49**

7-chloro 3,4-dihydrocarbostyril

Formule III: $R_1$ = 7-Cl; $R_2$ = $R_3$ = $R_4$ = H; X = CH; Y = $CH_2$

Selon le mode opératoire de l'exemple 46, mais à partir de 133,9 g de N-(méta-chlorophényl) β-chloropropionamide, on obtient 56 g de 7-chloro 3,4-dihydrocarbostyril sous forme de cristaux de point de fusion 160 - 165°C après recristallisation dans l'acétonitrile.

**Exemple 50**

2-para-fluorophénol 2H-1,4-benzothiazine-3 (4H)-one

Formule III: $R_1$ = $R_2$ = $R_3$ = H; $R_4$ = para-fluorophényl; X = CH; Y = S

Selon le mode opératoire de l'exemple 34, mais à partir de 20,7 g d'ortho-aminothiophénol, 6,7 g de soude et 43 g d'α-bromoparafluorophénylacétate d'éthyle, on obtient 34,7 g de 2-para-fluorophényl 2H-1,4-benzothiazine-3 (4H)-one sous forme de cristaux de point de fusion 215°C.

**Exemple 51**

2-ortho-fluorophényl 2H-1,4-benzothiazine-3 (4H)-one

Formule III: $R_1 = R_2 = R_3 = H$; $R_4$ = ortho-fluorophényl; X = CH; Y = S

Selon le mode opératoire de l'exemple 34, mais à partir de 20,7 g d'ortho-aminothiophénol, 6,7 g de soude et 43 g d'α-bromoorthofluorophénylacétate d'éthyle, on obtient 33,5 g de 2-ortho-fluorophényl 2H-1,4-benzothiazine-3 (4H)-one sous forme de cristaux de point de fusion 177°C.

**Exemple 52**

1-acétate d'éthyle-pyrido[2,3-b][1,4]thiazine-2 (3H)-one

Formule II: $R_1 = R_2 = R_3 = R_4 = H$; X = N; Y = S; Z = O; R' = $C_2H_5$

Une solution de 12 g de 1H-pyrido[2,3-b][1,4]thiazine-2 (3H)-one préparés à l'exemple 22 dans 75 ml de diméthylformamide est ajoutée goutte à goutte à une suspension de 3,5 g d'hydrure de sodium dans 30 ml de diméthylformamide. Après la fin de l'addition, on agite durant 30 min puis on ajoute goutte à goutte 9 ml de bromoacétate d'éthyle; quand l'addition est achevée, on agite encore durant 6 h à température ambiante, puis, après addition d'eau et de glace, on extrait à l'éther qu'on lave soigneusement à l'eau, sèche et qu'on évapore. Le résidu obtenu cristallise dans un mélange d'éther isopropylique et d'hexane. Les cristaux sont essorés et séchés. On obtient 9 g de 1-acétate d'éthyle-pyrido[2,3-b][1,4]thiazine-2 (3H)-one sous forme de cristaux de point de fusion 83°C.

**Exemple 53**

6-chloro 1-acétate d'éthyle-pyrido[2,3-b][1,4]thiazine-2 (3H)-one

Formule II: $R_1$ = 6-Cl; $R_2 = R_3 = H$; X = N; Y = S; Z = O; R' = $C_2H_5$

Selon le mode opératoire de l'exemple 52, mais à partir de 4,1 g de 6-chloro 1H-pyrido[2,3-b][1,4]thiazine-2 (3H)-one préparés à l'exemple 23, on obtient après cristallisation dans l'éther isopropylique 3,5 g de 6-chloro 1-acétate d'éthyle-pyrido[2,3-b][1,4]-thiazine-2 (3H))one sous forme de cristaux de point de fusion 105°C.

**Exemple 54**

3-méthyl 1-acétate d'éthyle-pyrido[2,3-b][1,4]thiazine-2 (3H)-one

Formule II: $R_1 = R_2 = R_3 = H$; $R_4 = CH_3$; X = N; Y = S; Z = O; R' = $C_2H_5$

Selon le mode opératoire de l'exemple 52, mais à partir de 15,7 g de 3-méthyl 1H-pyrido[2,3-b][1,4]thiazine-2 (3H)-one préparés à l'exemple 24, on obtient après recristallisation dans un mélange éther isopropylique-acétone 10 g de 3-méthyl 1-acétate d'éthyle-pyrido[2,3-b][1,4]thiazine-2 (3H)-one sous forme de cristaux de point de fusion 89°C.

**Exemple 55**

Chlorhydrate de 1-acétate d'éthyle-pyrido[2,3-b][1,4]pyrazine-2 (3H)-one

Formule II: $R_1 = R_2 = R_3 = R_4 = H$; X = Y = N; Z = O; R' = $C_2H_5$

Selon le mode opératoire de l'exemple 52, mais à partir de 12 g de 1H-pyrido[2,3-b][1,4]pyrazine-2 (3H)-one préparés à l'exemple 25, on obtient par extraction au chloroforme et cristallisation dans un mélange éther-éther de pétrole la base qui a un point de fusion de 142°C. Cette base est dissoute dans un mélange acétone-méthanol et additionnée d'éther chlorhydrique jusqu'à pH acide. Les cristaux formés essorés sont lavés à l'éther et séchés. On récupère ainsi 7,8 g de chlorhydrate de 1-acétate d'éthyle-pyrido[2,3-b[1,4]-pyrazine-2 (3H)-one sous forme de cristaux de point de fusion 207 - 210°C.

**Exemple 56**

6-trifluorométhyl 2H-1,4-benzothiazine-3-one 4-acétate d'éthyle

Formule II: $R_1$ = 6-$CF_3$; $R_2$ = $R_3$ = $R_4$ = H; X = CH; Y = S; Z = O; R' = $C_2H_5$

Selon le mode opératoire de l'exemple 52, mais à partir de 12 g de 6-trifluorométhyl 2H-1,4-benzothiazine-3 (4H)-one préparés à l'exemple 26, on obtient, après cristallisation dans le pentane, 8 g de 6-trifluorométhyl 2H-1,4-benzothiazine-3-one 4-acétate d'éthyle sous forme de cristaux de point de fusion 60° C.

**Exemple 57**

7-fluoro 2H-1,4-benzothiazine-3-one 4-acétate d'éthyle

Formule II: $R_1$ = 7-F; $R_2$ = $R_3$ = $R_4$ = H; X = CH; Y = S; Z= O; R' = $C_2H_5$

Selon le mode opératoire de l'exemple 52, mais à partir de 20 g de 7-fluoro 2H-1,4-benzothiazine-3 (4H)-one préparés à l'exemple 27, on obtient, après cristallisation dans un mélange éther-pentane, 19 g de 7-fluoro 2H-1,4-benzothiazine-3-one 4-acétate d'éthyle sous forme de cristaux de point de fusion 85° C.

**Exemple 58**

6-fluoro 2H-1,4-benzothiazine-3-one 4-acétate d'éthyle

Formule II: $R_1$ = 6-F; $R_2$ = $R_3$ = $R_4$ = H; X = CH; Y = S; Z = O; R' = $C_2H_5$

Selon le mode opératoire de l'exemple 52, mais à partir de 43 g de 6-fluoro 2H-1,4-benzothiazine-3 (4H)-one préparés à l'exemple 28, on obtient après cristallisation dans un mélange acétone-pentane 36 g de 6-fluoro 2H-1,4-benzothiazine-3-one 4-acétate d'éthyle sous forme de cristaux de point de fusion 80° C.

**Exemple 59**

8-chloro 2H-1,4-benzothiazine-3-one 4-acétate d'éthyle

Formule II: $R_1$ = 8-Cl; $R_2$ = $R_3$ = $R_4$ = H; X = CH = Y = S; Z = O; R' = $C_2H_5$

Selon le mode opératoire de l'exemple 52, mais à partir de 12 g de 8-chloro 2H-1,4-benzothiazine-3 (4H)-one préparés à l'exemple 29, on récupère 14 g de 8-chloro 2H-1,4-benzothiazine-3-one 4-acétate d'éthyle sous forme d'une huile qu'on peut utiliser brute pour l'étape suivante.

**Exemple 60**

7-chloro 2H-1,4-benzothiazine-3-one 4-acétate d'éthyle

Formule II: $R_1$ = 7-Cl; $R_2$ = $R_3$ = $R_4$ = H; X = CH; Y = S; Z = O; R' = $C_2H_5$

Selon le mode opératoire de l'exemple 52, mais à partir de 23 g de 7-chloro 2H-1,4-benzothiazine-3 (4H)-one préparés à l'exemple 30, on obtient 32 g de 7-chloro 2H-1,4-benzothiazine-3-one 4-acétate d'éthyle sous forme de cristaux de point de fusion 75 - 78° C.

**Exemple 61**

6-méthoxy 2H-1,4-benzothiazine-3-one 4-acétate d'éthyle

Formule II: $R_1$ = 6-$OCH_3$; $R_2$ = $R_3$ = $R_4$ = H; X = CH; Y = S; Z = O; R' = $C_2H_5$

Selon le mode opératoire de l'exemple 52, mais à partir de 26,9 g de 6-méthoxy 2H-1,4-benzothiazine-3 (4H)-one préparés à l'exemple 31, on obtient après cristallisation dans l'éther 24,5 g de 6-méthoxy 2H-1,4-benzothiazine-3-one 4-acétate d'éthyle sous forme de cristaux de point de fusion 84 - 87° C.

**Exemple 62**

6-trifluorométhyl 7-chloro 2H-1,4-benzothiazine-3-one 4-acétate d'éthyle

Formule II: $R_1 = 6\text{-}CF_3$; $R_2 = 7\text{-}Cl$; $R_3 = R_4 = H$; X = CH; Y = S; Z = O; $R' = C_2H_5$

Selon le mode opératoire de l'exemple 52, mais à partir de 15,5 g de 6-trifluorométhyl-7-chloro 2H-1,4-benzothiazine-3 (4H)-one préparés à l'exemple 32, on obtient après cristallisation dans le pentane 13 g de 6-trifluorométhyl 2-chloro 2H-1,4-benzothiazine-3-one 4-acétate d'éthyle sous forme de cristaux de point de fusion 93° C.

**Exemple 63**

2H-1,4-benzothiazine-3-one 4-acétate d'éthyle

Formule II: $R_1 = R_2 = R_3 = R_4 = H$; X = CH; Y = S; Z = O; $R' = C_2H_5$

Selon le mode opératoire de l'exemple 52, mais à partir de 23 g de 2H-1,4-benzothiazine-3 (4H)-one préparés à l'exemple 33, on obtient après cristallisation dans l'éther isopropylique 21,8 g de 2H-1,4-benzothiazine-3-one 4-acétate d'éthyle sous forme de cristaux de point de fusion 43 - 44° C.

**Exemple 64**

2-méthyl 2H-1,4-benzothiazine-3-one 4-acétate d'éthyle

Formule II: $R_1 = R_2 = R_3 = H$; $R_4 = CH_3$; X = CH; Y = S; Z = O; $R' = C_2H_5$

Selon le mode opératoire de l'exemple 52, mais à partir de 34 g de 2-méthyl 2H-1,4-benzothiazine-3 (4H)-one préparés à l'exemple 34, on obtient après cristallisation dans le pentane 39,6 g de 2-méthyl 2H-1,4-benzothiazine-3-one 4-acétate d'éthyle sous forme de cristaux de point de fusion 70 - 72° C.

**Exemple 65**

2-phényl 2H-1,4-benzothiazine-3-one 4-acétate d'éthyle

Formule II: $R_1 = R_2 = R_3 = H$; $R_4$ = phényle; X = CH; Y = S; Z = O; $R' = C_2H_5$

Selon le mode opératoire de l'exemple 52, mais à partir de 45,5 g de 2-phényl 2H-1,4-benzothiazine-3 (4H)-one préparés à l'exemple 35, on obtient 38 g de 2-phényl 2H-1,4-benzothiazine-3-one 4-acétate d'éthyle sous forme d'une huile qu'on peut utiliser brute pour l'étape suivante.

**Exemple 66**

6-chloro 2H-1,4-benzothiazine-3-one 4-acétate d'éthyle

Formule II: $R_1 = 6\text{-}Cl$; $R_2 = R_3 = R_4 = H$; X = CH; Y = S; Z = O; $R' = C_2H_5$

Selon le mode opératoire de l'exemple 52, mais à partir de 27,3 g de 6-chloro 2H-1,4-benzothiazine-3 (4H)-one préparés à l'exemple 36, on récupère après recristallisation dans le pentane, 32 g de 6-chloro 2H-1,4-benzothiazine-3-one 4-acétate d'éthyle sous forme de cristaux de point de fusion 72° C.

**Exemple 67**

6-fluoro 2-méthyl 2H-1,4-benzothiazine-3-one 4-acétate d'éthyle

Formule II: $R_1$ = 6-F; $R_3$ = $CH_3$; $R_2$ = $R_4$ = H; X = CH; Y = S; Z = O; R' = $C_2H_5$

Selon le mode opératoire de l'exemple 52, mais à partir de 20 g de 6-fluoro 2-méthyl 2H-1,4-benzothiazine-3 (4H)-one préparés à l'exemple 37, on obtient après cristallisation dans un mélange éther isopropylique-pentane, 13 g de 6-fluoro 2-méthyl 2H-1,4-benzothiazine-3-one 4-acétate d'éthyle sous forme de cristaux de point de fusion 92° C.

**Exemple 68**

6-chloro 2-méthyl 2H-1,4-benzothiazine-3-one 4-acétate d'éthyle

Formule II: $R_1$ = 6-Cl; $R_3$ = $CH_3$; $R_2$ = $R_4$ = H; X = CH; Y = S; Z = O; R' = $C_2H_5$

Selon le mode opératoire de l'exemple 52, mais à partir de 19 g de 6-chloro 2-méthyl 2H-1,4-benzothiazine-3 (4H)-one préparés à l'exemple 38, on récupère après cristallisation dans un mélange éther isopropylique-pentane, 24 g de 6-chloro 2-méthyl 2H-1,4-benzothiazine-3-one, 4-acétate d'éthyle sous forme de cristaux de point de fusion 105° C.

**Exemple 69**

6-chloro 2-phényl 2H-1,4-benzothiazine-3-one 4-acétate d'éthyle

Formule II: $R_1$ = 6-Cl; $R_3$ = phényl; $R_2$ = $R_4$ = H; X = CH; Y = S; Z = O; R' = $C_2H_5$

Selon le mode opératoire de l'exemple 52, mais à partir de 6,3 g de 6-chloro 2-phényl 2H-1,4-benzothiazine-3 (4H)-one préparés à l'exemple 39, on récupère 8 g de 6-chloro 2-phényl 2H-1,4-benzothiazine-3-one 4-acétate d'éthyle sous forme d'une huile qu'on peut utiliser brute pour l'étape suivante:

**Exemple 70**

2,2-diméthyl 2H-1,4-benzothiazine-3-one 4-acétate d'éthyle

Formule II: $R_1$ = $R_2$ = H; $R_3$ = $R_4$ = $CH_3$; X = CH; Y = S; Z = O; R' = $C_2H_5$

Sélon le mode opératoire de l'exemple 52, mais à partir de 40 g de 2,2-diméthyl 2H-1,4-benzothiazine-3 (4H)-one préparés à l'exemple 40, on récupère après cristallisation dans le pentane 50,1 g de 2,2-diméthyl-2H-1,4-benzothiazine-3-one 4-acétate d'éthyle sous forme de cristaux de point de fusion 60° C.

**Exemple 71**

2-para-chlorophényl 2H-1,4-benzothiazine-3-one 4-acétate d'éthyle

Formule II: $R_1$ = $R_2$ = $R_3$ = H; $R_4$ = parachlorophényl; X = CH; Y = S; Z = O; R' = $C_2H_5$

Selon le mode opératoire de l'exemple 52, mais à partir de 37,8 g de 2-para-chlorophényl 2H-1,4-benzothiazine-3 (4H)-one préparés à l'exemple 41, on obtient après cristallisation dans un mélange éther isopropylique-pentane-isopropanol 25 g de 2-parachlorophényl 2H-1,4-benzothiazine-3-one 4-acétate d'éthyle sous forme de cristaux de point de fusion 118° C.

**Exemple 72**

2-ortho-chlorophényl 2H-1,4-benzothiazine-3-one 4-acétate d'éthyle

Formule II: $R_1 = R_2 = R_3 = H$; $R_4$ = ortho-chlorophényl; X = CH; Y = S; Z = O; R' = $C_2H_5$

Selon le mode opératoire de l'exemple 52, mais à partir de 37 g de 2-ortho-chlorophényl 2H-1,4-benzothiazine-3 (4H)-one préparés à l'exemple 42, on obtient 44,3 g de 2-ortho-chlorophényl 2H-1,4-benzothiazine-3-one 4-acétate d'éthyle sous forme d'une huile qu'on peut utiliser brute pour l'étape suivante.

**Exemple 73**

6,7-dichloro 2H-1,4-benzothiazine-3-one 4-acétate d'éthyle

Formule II: $R_1$ = 6-Cl; $R_2$ = 7Cl; $R_3 = R_4 = H$; X = CH; Y = S; Z = O; R' = $C_2H_5$

Selon le mode opératoire de l'exemple 52, mais à partir de 4,5 g de 6,7-dichloro 2H-1,4-benzothiazine-3 (4H)-one préparés à l'exemple 42, on obtient après cristallisation dans l'éther isopropylique 4 g de 6,7-dichloro 2H-1,4-benzothiazine-3-one 4-acétate d'éthyle sous forme de cristaux de point de fusion 160° C.

**Exemple 74**

2-méthyl 2-(2'-pyridyl) 2H-1,4-benzothiazine-3-one 4-acétate d'éthyle

Formule II: $R_1 = R_2 = H$; $R_3 = CH_3 = R_4$ = 2'-pyridyl; X = CH; Y = S; Z = O; R' = $C_2H_5$

Selon le mode opératoire de l'exemple 52, mais à partir de 9,5 g de 2-méthyl 2-(2'-pyridyl) 2H-1,4-benzothiazine-3 (4H)-one préparés à l'exemple 44, on obtient 12 g de 2-méthyl 2-(2'-pyridyl) 2H-1,4-benzothiazine-3-one 4-acétate d'éthyle sous forme d'une huile qu'on peut utiliser brute pour l'étape suivante.

**Exemple 75**

6-fluoro 2H-1,4-benzoxazine-3-one 4-acétate d'éthyle

Formule II: $R_1$ = 6-F; $R_2 = R_3 = R_4 = H$; X = CH; Y = Z = O; R' = $C_2H_5$

Selon le mode opératoire de l'exemple 52, mais à partir de 27,3 g de 6-fluoro 2H-1,4-benzoxazine-3 (4H)-one préparés à l'exemple 45, on récupère 32 g de 6-fluoro 2H-1,4-benzoxazine-3-one 4-acétate d'éthyle sous forme de cristaux de point de fusion 73° C.

**Exemple 76**

6-fluoro 3,4-dihydrocarbostyril 1-acétate d'éthyle

Formule II: $R_1$ = 6-F; $R_2 = R_3 = R_4 = H$; X = CH; Y = $CH_2$; Z = O; R' = $C_2H_5$

Selon le mode opératoire de l'exemple 52, mais à partir de 12,8 g de 6-fluoro 3,4-dihydrocarbostyril préparés à l'exemple 46, on récupère après cristallisation dans un mélange éther-pentane, 12,5 g de 6-fluoro 3,4-dihydrocarbostyril 1-acétate d'éthyle sous forme de cristaux de point de fusion 82 - 86° C.

**Exemple 77**

6-chloro 3,4-dihydrocarbostyril 1-acétate d'éthyle

Formule II: $R_1$ = 6-Cl; $R_2 = R_3 = R_4 = H$; X = CH; Y = $CH_2$; Z = O; R' = $C_2H_5$

Selon le mode opératoire de l'exemple 52, mais à partir de 33 g de 6-chloro 3,4-dihydrocarbostyril préparés à l'exemple 47, on récupère après cristallisation dans un mélange éther-pentane 30,7 g de 6-chloro 3,4-dihydrocarbostyril 1-acétate d'éthyle sous forme de cristaux de point de fusion 72 - 75° C.

**Exemple 78**

7-fluoro 3,4-dihydrocarbostyril 1-acétate d'éthyle

Formule II: $R_1$ = 7-F; $R_2$ = $R_3$ = $R_4$ = H; X = CH; Y= $CH_2$; Z = O; R' = $C_2H_5$

Selon le mode opératoire de l'exemple 52, mais à partir de 30,8 g de 7-fluoro 3,4-dihydrocarbostyril préparés à l'exemple 48, on récupère après cristallisation dans le pentane 36,3 g de 7-fluoro 3,4-dihydrocarbostyril 1-acétate d'éthyle sous forme de cristaux de point de fusion 75 - 78° C.

**Exemple 79**

7-chloro 3,4-dihydrocarbostyril 1-acétate d'éthyle

Formule II: $R_1$ = 7-Cl; $R_2$ = $R_3$ = $R_4$ = H; X = CH; Y = $CH_2$; Z = O; R' = $C_2H_5$

Selon le mode opératoire de l'exemple 52, mais à partir de 40 g de 7-chloro 3,4-dihydrocarbostyril préparés à l'exemple 49, on récupère après cristallisation dans le mélange éther-pentane 39,8 g de 7-chloro 3,4-dihydrocarbostyril 1-acétate d'éthyle sous forme de cristaux de point de fusion 79 - 83° C.

**Exemple 80**

2-para-fluorophényl 2H-1,4-benzothiazine-3-one 4-acétate d'éthyle

Formule II: $R_1$ = $R_2$ = $R_3$ = H; $R_4$ = para-fluorophényl; X = CH; Y = S; Z = O; R' = $C_2H_5$

Selon le mode opératoire de l'exemple 52, mais à partir de 34,7 g de 2-para-fluorophényl 2H-1,4-benzothiazine-3 (4H)-one préparés à l'exemple 50, on obtient 35 g de 2-para-fluorophényl 2H-1,4-benzothiazine-3-one 4-acétate d'éthyle sous forme d'une huile qu'on peut utiliser brute pour l'étape suivante.

**Exemple 81**

2-ortho-fluorophényl 2H-1,4-benzothiazine-3-one 4-acétate d'éthyle

Formule II: $R_1$ = $R_2$ = $R_3$ = H; $R_4$ = ortho-fluorophényl; X = CH; Y = S; Z = O; R' = $C_2H_5$

Selon le mode opératoire de l'exemple 52, mais à partir de 33 g de 2-ortho-fluorophényl 2H-1,4-benzothiazine-3 (4H)-one préparés à l'exemple 51, on obtient 37,4 g de 2-ortho-fluorophényl-2H-1,4-benzothiazine-3-one 4-acétate d'éthyle sous forme de cristaux de point de fusion 118° C.

**Exemple 82**

7-chloro 2-méthyl 2H-1,4-benzothiazine-3 (4H)-one

Formule III: $R_1$ = 7-Cl; $R_3$ = $CH_3$; $R_2$ = $R_4$ = H; X = CH; Y = S

Selon le mode opératoire de l'exemple 28, mais en utilisant 72 g de 5-chloro 2-nitrophénylthio α-méthylacétate d'éthyle, préparés à l'exemple 21, on obtient 26,8 g de 7-chloro 2-méthyl 2H-1,4-benzothiazine-3 (4H)-one sous forme de cristaux de point de fusion 200° C.

**Exemple 83**

7-chloro 2-méthyl 2H-1,4-benzothiazine-3-one 4-acétate d'éthyle

Formule II: $R_1$ = 7-Cl; $R_3$ = $CH_3$; $R_2$ = $R_4$ = H; X = CH; Y = S; Z = O; R' = $C_2H$

Selon le mode opératoire de l'exemple 52, mais à partir de 19,7 g de 7-chloro 2-méthyl 2H-1,4-benzothiazine-3 (4H)-one préparés à l'exemple 82, on obtient après cristallisation dans l'éther 20,7 g de 7-chloro 2-méthyl 2H-1,4-benzothiazine-3-one 4-acétate d'éthyle sous forme de cristaux de point de fusion 112 - 113° C.

**Exemple 84**

7-fluoro 2H-1,4-benzothiazine-3-thione 4-acétate d'éthyle

Formule II: $R_1$ = 7-F; $R_2$ = $R_3$ = $R_4$ = H; X = CH; Y = Z = S; R' = $C_2H_5$

On agite durant 15 h à température ambiante une solution de 19 g de 7-fluoro 2H-1,4-benzothiazine-3-one 4-acétate d'éthyle, préparés à l'exemple 57, dans 200 ml de chloroforme contenant 19 g de $P_2S_5$. La solution est ensuite filtrée puis concentrée sous vide et le résidu est filtré sur silicagel. Par élution au benzène puis recristallisation des cristaux obtenus dans l'hexane, on obtient 11,2 g de 7-fluoro 2H-1,4-benzothiazine-3-thione 4-acétate d'éthyle sous forme de cristaux de point de fusion 87° C.

**Exemple 85**

6-fluoro 2H-1,4-benzothiazine-3-thione 4-acétate-d'éthyle

Formule II: $R_1$ = 6-F, $R_2$ = $R_3$ = $R_4$ = H; X = CH, Y = Z = S; R' = $C_2H_5$

Selon le mode opératoire de l'exemple 84, mais à partir de 15 g de 6-fluoro 2H-1,4-benzothiazine-3-one 4-acétate d'éthyle préparés à l'exemple 58, on obtient après filtration sur silicagel, élution au benzène et cristallisation dans le mélange éther isopropylique-pentane, 7,4 g de 6-fluoro 2H-1,4-benzothiazine-3-thione 4-acétate d'éthyle sous forme de cristaux de point de fusion 66° C.

**Exemple 86**

7-chloro 2H-1,4-benzothiazine-3-thione 4-acétate d'éthyle

Formule II: $R_1$ = 7C1; $R_2$ = $R_3$ = $R_4$ = H; X = CH; Y = Z = S; R' = $C_2H_5$

Selon le mode opératoire de l'exemple 84, mais à partir de 32 g de 7-chloro 2H-1,4-benzothiazine-3-one 4-acétate d'éthyle préparés à l'exemple 60, on récupère après cristallisation dans le pentane, 20,5 g de 7-chloro 2H-1,4-benzothiazine-3-thione 4-acétate d'éthyle sous forme de cristaux de point de fusion 98° C.

**Exemple 87**

6-méthoxy 2H-1,4-benzothiazine-3-thione 4-acétate d'éthyle

Formule II: $R_1$ = 6-$OCH_3$; $R_2$ = $R_3$ = $R_4$ = H; X = CH; Y = Z = S; R' = $C_2H_5$

Selon le mode opératoire de l'exemple 84, mais à partir de 12 g de 6-méthoxy 2H-1,4-benzothiazine-3-one 4-acétate d'éthyle préparés à l'exemple 61, on récupère 8 g de 6-méthoxy 2H-1,4-benzothiazine-3-thione-4-acétate d'éthyle.

**Exemple 88**

6-trifluorométhyl 7-chloro 2H-1,4-benzothiazine-3-thione 4-acétate d'éthyle

Formule II: $R_1$ = 6-$CF_3$; $R_2$ = 7-Cl; $R_3$ = $R_4$ = H; X = CH; Y = Z = S; R' = $C_2H_5$

Selon le mode opératoire de l'exemple 84, mais à partir de 13 g de 6-trifluorométhyl 7-chloro 2H-1,4-benzothiazine-3-one 4-acétate d'éthyle préparés à l'exemple 62, on récupère après cristallisation dans le pentane 5,1 g de 6-trifluorométhyl 7-chloro 2H-1,4-benzothiazine-3-thione 4-acétate d'éthyle sous forme de cristaux de point de fusion 83°C.

**Exemple 89**

2-phényl 2H-1,4-benzothiazine-3-thione 4-acétate d'éthyle

Formule II: $R_1$ = $R_2$ = $R_3$ = H; $R_4$ = phényle; X = CH; Y = Z = S; R' = $C_2H_5$

Selon le mode opératoire de l'exemple 84, mais à partir de 23 g de 2-phényl 2H-1,4-benzothiazine-3-one 4-acétate d'éthyle préparés à l'exemple 65, on récupère 9 g de 2-phényl 2H-1,4-benzothiazine-3-thione 4-acétate d'éthyle sous forme de cristaux de point de fusion 135°C.

**Exemple 90**

6-trifluorométhyl 2H-1,4-benzothiazine-3-thione 4-acétate d'éthyle

Formule II: $R_1$ = 6-$CF_3$; $R_2$ = $R_3$ = $R_4$ = H; X = CH; Y = Z = S; R' = $C_2H_5$

Selon le mode opératoire de l'exemple 84, mais à partir de 31 g de 6-trifluorométhyl 2H-1,4-benzothiazine-3-one 4-acétate d'éthyle préparés à l'exemple 56, on obtient après recristallisation dans l'isopropanol 14 g de 6-trifluorométhyl 2H-1,4-benzothiazine-3-thione 4-acétate d'éthyle sous forme de cristaux de point de fusion 110°C.

**Exemple 91**

2-méthyl 2H-1,4-benzothiazine-3-thione 4-acétate d'éthyle

Formule II: $R_1$ = $R_2$ = $R_3$ = H; $R_4$ = $CH_3$; X = CH; Y = Z = S; R' = $C_2H_5$

On porte à 80°C durant 5 h une solution de 20 g de 2-méthyl 2H-1,4-benzothiazine-3-one 4-acétate d'éthyle préparés à l'exemple 64 dans 100 ml de diméthoxyéthane en présence de 20 g de réactif de Lawesson. Le mélange réactionnel est ensuite concentré sous vide, le résidu repris à chaud par 400 ml de cyclohexane et, après filtration, le cyclohexane est évaporé sous vide et le résidu obtenu est filtré sur gel de silice. Par élution au toluène, on récupère 11 g de 2-méthyl 2H-1,4-benzothiazine-3-thione 4-acétate d'éthyle sous forme de cristaux de point de fusion 65°C.

**Exemple 92**

2H-1,4-benzothiazine-3-thione 4-acétate d'éthyle

Formule II: $R_1$ = $R_2$ = $R_3$ = $R_4$ = H; X = CH; Y = Z = S; R' = $C_2H_5$

Selon le mode opératoire de l'exemple 84, mais à partir de 35 g de 2H-1,4-benzothiazine-3-one 4-acétate d'éthyle préparés à l'exemple 63, on obtient après cristallisation dans le mélange pentane-éther isopropylique 21 g de 2H-1,4-benzothiazine-3-thione 4-acétate d'éthyle sous forme de cristaux de point de fusion 50°C.

**Exemple 93**

6-chloro 2H-1,4-benzothiazine-3-thione 4-acétate d'éthyle

Formule II: $R_1$ = 6-Cl; $R_2$ = $R_3$ = $R_4$ = H; X = CH; Y = Z = S; R' = $C_2H_5$

Selon le mode opératoire de l'exemple 84, mais à partir de 23 g de 6-chloro 2H-1,4-benzothiazine-3-one 4-acétate d'éthyle préparés à l'exemple 66, on obtient après cristallisation dans le mélange éther isopropylique-pentane 11,5 g de 6-chloro 2H-1,4-benzothiazine-3-thione 4-acétate d'éthyle sous forme de cristaux de point de fusion 64° C.

**Exemple 94**

6-fluoro 2-méthyl 2H-1,4-benzothiazine-3-thione 4-acétate d'éthyle

Formule II: $R_1$ = 6-F; $R_3$ = $CH_3$; $R_2$ = $R_4$ = H; X = CH; Y = Z = S; R' = $C_2H_5$

Selon le mode opératoire de l'exemple 84, mais à partir de 11 g de 6-fluoro 2-méthyl 2H-1,4-benzothiazine-3-one 4-acétate d'éthyle préparés à l'exemple 67, on obtient 6 g de 6-fluoro 2-méthyl 2H-1,4-benzothiazine-3-thione 4-acétate d'éthyle sous forme de cristaux de point de fusion 91° C.

**Exemple 95**

6-chloro 2-méthyl 2H-1,4-benzothiazine-3-thione 4-acétate d'éthyle

Formule II: $R_1$ = 6-Cl; $R_2$ = $CH_3$; $R_2$ = $R_4$ = H; X = CH; Y = Z = S; R' = $C_2H_5$

Selon le mode opératoire de l'exemple 84, mais à partir de 17 g de 6-chloro 2-méthyl 2H 1,4-benzothiazine-3-one 4-acétate d'éthyle préparés à l'exemple 68, on obtient après cristallisation dans le pentane 9,5 g de 6-chloro 2-méthyl 2H 1,4-benzothiazine-3-thione 4-acétate d'éthyle sous forme de cristaux de point de fusion 55° C.

**Exemple 96**

6-chloro 2-phényl 2H-1,4-benzothiazine-3-thione 4-acétate d'éthyle

Formule II: $R_1$ = 6-Cl; $R_3$ = phényle; $R_2$ = $R_4$ = H; X = CH; Y = Z = S; R' = $C_2H_5$

Selon le mode opératoire de l'exemple 84, mais à partir de 21 g de 6-chloro 2-phényl 2H-1,4-benzothiazine-3-one 4-acétate d'éthyle préparés à l'exemple 69, on obtient après cristallisation dans l'éther 11 g de 6-chloro 2-phényl 2H-1,4-benzothiazine-3-thione 4-acétate d'éthyle sous forme de cristaux de point de fusion 148° C.

**Exemple 97**

2,2-diméthyl 2H-1,4-benzothiazine-3-thione 4-acétate d'éthyle

Formule II: $R_1$ = $R_2$ = H; $R_3$ = $R_4$ = $CH_3$; X = CH; Y = Z = S; R' = $C_2H_5$

Selon le mode opératoire de l'exemple 84, mais à partir de 25 g de 2,2-diméthyl 2H-1,4-benzothiazine-3-one 4-acétate d'éthyle préparés à l'exemple 70, on obtient après cristallisation dans le pentane, 11,2 g de 2,2-diméthyl 2H-1,4-benzothiazine-3-thione 4-acétate d'éthyle sous forme de cristaux de point de fusion 82 - 86° C.

**Exemple 98**

2-para-chlorophényl 2H-1,4-benzothiazine-3-thione 4-acétate d'éthyle

Formule II: $R_1 = R_2 = R_3 = H$; $R_4$= para-chlorophényl; X = CH; Y = Z = S; R' = $C_2H_5$

Selon le mode opératoire de l'exemple 84, mais à partir de 17,5 g de 2-para-chlorophényl 2H-1,4-benzothiazine-3-one 4-acétate d'éthyle préparés à l'exemple 71, on obtient après cristallisation dans un mélange éther isopropylique-pentane, 11,2 g de 2-parachlorophényl 2H-1,4-benzothiazine-3-thione 4-acétate d'éthyle sous forme de cristaux de point de fusion 94° C.

**Exemple 99**

6,7-dichloro 2H-1,4-benzothiazine-3-thione 4-acétate d'éthyle

Formule II: $R_1$ = 6-Cl; $R_2$ = 7-Cl: $R_3 = R_4 = H$; X = CH; Y = Z = S; R' = $C_2H_5$

Selon le mode opératoire de l'exemple 84, mais à partir de 4 g de 6,7-dichloro 2H-1,4-benzothiazine-3-one 4-acétate d'éthyle préparés à l'exemple 73, on obtient après cristallisation dans le pentane 2,4 g de 6,7-dichloro 2H-1,4-benzothiazine-3-thione 4-acétate d'éthyle sous forme de cristaux de point de fusion 123° C.

**Exemple 100**

7-chloro 2-méthyl 2H-1,4-benzothiazine-3-thione 4-acétate d'éthyle

Formule II: $R_1$ = Cl; $R_3 = CH_3$; $R_2 = R_4 = H$; X = CH; Y = Z = S; R' = $C_2H_5$

Selon le mode opératoire de l'exemple 84, mais à partir de 11,5 g de 7-chloro 2-méthyl 2H-1,4-benzothiazine-3-one 4-acétate d'éthyle préparés à l'exemple 83, on obtient 9,2 g de 7-chloro 2-méthyl 2H-1,4-benzothiazine-3-thione 4-acétate d'éthyle.

**Exemple 101**

2-ortho-chlorophényl 2H-1,4-benzothiazine-3-thione 4-acétate d'éthyle

Formule II: $R_1 = R_2 = R_3 = H$; $R_4$ = ortho-chlorophényl; X = CH; Y = Z = S; R' = $C_2H_5$

Selon le mode opératoire de l'exemple 84, mais à partir de 32 g de 2-ortho-chlorophényl 2H-1,4-benzothiazine-3-one 4-acétate d'éthyle préparés à l'exemple 72, on obtient après recristallisation dans l'acétonitrile 18 g de 2-ortho-chlorophényl 2H-1,4-benzothiazine-3-thione 4-acétate d'éthyle sous forme de cristaux de point de fusion 154° C.

**Exemple 102**

6-fluoro 2H-1,4-benzoxazine-3-thione 4-acétate d'éthyle

Formule II: $R_1$ = 6-F; $R_2 = R_3 = R_4 = H$; X = CH; Y = O; Z = S; R' = $C_2H_5$

Selon le mode opératoire de l'exemple 84, mais à partir de 22 g de 6-fluoro 2H-1,4-benzoxazine-3-one 4-acétate d'éthyle préparés à l'exemple 75, on obtient après cristallisation dans le mélange éther-pentane 13,8 g de 6-fluoro 2H-1,4-benzoxazine-3-thione 4-acétate d'éthyle sous forme de cristaux de point de fusion 52° C.

**Exemple 103**

6-fluoro 3,4-dihydroquinoline 2-thione 1-acétate d'éthyle

Formule II: $R_1$ = 6-F; $R_2$ = $R_3$ = $R_4$ = H; X = CH; Y = $CH_2$; Z = S, R' = $C_2H_5$

Selon le mode opératoire de l'exemple 84, mais à partir de 17 g de 6-fluoro 3,4-dihydrocarbostyril 1-acétate d'éthyle préparés à l'exemple 76, on récupère 6,5 g de 6-fluoro 3,4-dihydroquinoline 2-thione 1-acétate d'éthyle sous forme de cristaux de point de fusion 80 - 82° C.

**Exemple 104**

6-chloro 3,4-dihydroquinoline 2-thione 1-acétate d'éthyle

Formule II: $R_1$ = 6-Cl; $R_2$ = $R_3$ = $R_4$ = H; X = CH; Y = $CH_2$; Z = S; R' = $C_2H_5$

Selon le mode opératoire de l'exemple 84, mais à partir de 20 g de 6-chloro 3,4-dihydrocarbostyril 1-acétate d'éthyle préparés à l'exemple 77, on récupère 9,4 g de 6-chloro 3,4-dihydroquinoline 2-thione 1-acétate d'éthyle sous forme de cristaux de point de fusion 84 - 87° C.

**Exemple 105**

7-fluoro 3,4-dihydroquinoline 2-thione 1-acétate d'éthyle

Formule II: $R_1$ = 7-F; $R_2$ = $R_3$ = $R_4$ = H; X = CH; Y = $CH_2$; Z = S; R' = $C_2H_5$

Selon le mode opératoire de l'exemple 84, mais à partir de 26 g de 7-fluoro 3,4-dihydrocarbostyril 1-acétate d'éthyle préparés à l'exemple 78, on récupère 15,7 g de 7-fluoro 3,4-dihydroquinoline 2-thione 1-acétate d'éthyle sous forme de cristaux de point de fusion 76 - 79° C.

**Exemple 106**

7-chloro 3,4-dihydroquinoline 2-thione 1-acétate d'éthyle

Formule II: $R_1$ = 7-Cl; $R_2$ = $R_3$ = $R_4$ = H; X = CH; Y = $CH_2$; Z = S; R' = $C_2H_5$

Selon le mode opératoire de l'exemple 84, mais à partir de 25,5 g de 7-chloro 3,4-dihydrocarbostyril 1-acétate d'éthyle préparés à l'exemple 79, on récupère 12 g de 7-chloro 3,4-dihydroquinoline 2-thione 1-acétate d'éthyle sous forme de cristaux de point de fusion 68 - 72° C.

**Exemple 107**

3-méthyl 1-acétate d'éthyle-pyrido[2,3-b][1,4]thiazine-2 (3H)-thione

Formule II: $R_1$ = $R_2$ = $R_3$ = H; $R_4$ = $CH_3$; X = N; Y = Z = S; R' = $C_2H_5$

Selon le mode opératoire de l'exemple 84, mais à partir de 10 g de 3-méthyl 1-acétate d'éthyle-pyrido[2,3-b][1,4]thiazine-2 (3H)-one préparés à l'exemple 54, on récupère 3 g de 3-méthyl 1-acétate d'éthyle-pyrido[2,3-b][1,4]thiazine-2 (3H)-thione sous forme de cristaux de point de fusion 100° C.

**Exemple 108**

2-para-fluorophényl 2H-1,4-benzothiazine-3-thione 4-acétate d'éthyle

Formule II: $R_1$ = $R_2$ = $R_3$ = H; $R_4$ = para-fluorophényl; X = CH; Y = Z = S; R' = $C_2H_5$

Selon le mode opératoire de l'exemple 84, mais à partir de 23 g de 2-para-fluorophényl 2H-1,4-benzothiazine-3-one 4-acétate d'éthyle préparés à l'exemple 80, on récupère 17 g de 2-para-fluorophényl-2H-1,4-benzothiazine-3-thione 4-acétate d'éthyle.

**Exemple 109**

2-ortho-fluorophényl 2H-1,4-benzothiazine-3-thione 4-acétate d'éthyle

Formule II: $R_1 = R_2 = R_3 = H$; $R_4$ = ortho-fluorophényl; X = CH; Y = Z = S; R' = $C_2H_5$

Selon le mode opératoire de l'exemple 84, mais à partir de 25 g de 2-ortho-fluorophényl 2H-1,4-benzothiazine-3-one 4-acétate d'éthyle préparés à l'exemple 81, on récupère 19 g de 2-ortho-fluorophényl 2H-1,4-benzothiazine-3-thione 4-acétate d'éthyle sous forme de cristaux de point de fusion 174° C.

**Exemple 110**

1-acide acétique-pyrido[2,3-b]thiazine-2 (3H)-one

Formule I: $R_1 = R_2 = R_3 = R_4 = H$; X = N; Y = S; Z = O

On porte 10 min à 50° C une solution de 9 g de 1-acétate d'éthyle-pyrido[2,3-b]thiazine-2 (3H)-one préparés à l'exemple 52 dans 30 ml de méthanol contenant 1,5 g de soude dissoute dans 20 ml d'eau. Le mélange réactionnel est ensuite traité au charbon actif, puis filtré et acidifié à froid par de l'acide chlorhydrique dilué, concentré sous vide de moitié puis additionné d'eau et de glace. Les cristaux formés sont essorés, lavés 2 fois avec 10 ml d'eau puis à l'acétone et séchés. On récupère ainsi 5,5 g de 1-acide acétique-pyrido[2,3-b]thiazine-2 (3H)-one sous forme de cristaux de point de fusion 252 - 255° C.

**Exemple 111**

6-chloro 1-acide acétique-pyrido[2,3-b]thiazine-2 (3H)-one

Formule I: $R_1$ = 6-Cl; $R_2 = R_3 = R_4 = H$; X = N; Y = S; Z = O

Selon le mode opératoire de l'exemple 110, mais en utilisant 3,5 g de 6-chloro 1-acétate d'éthyle-pyrido[2,3-b]thiazine-2 (3H)-one préparés à l'exemple 53, on obtient 2 g de 6-chloro 1-acide acétique-pyrido[2,3-b]thiazine-2 (3H)-one sous forme de cristaux de point de fusion 195 - 200° C.

**Exemple 112**

3-méthyl 1-acide acétique-pyrido[2,3-b]-thiazine-2 (3H)-one

Formule I: $R_1 = R_2 = R_3 = H$; $R_4 = CH_3$; X = N; Y = S; Z = O

Selon le mode opératoire de l'exemple 110, mais en utilisant 7 g de 3-méthyl 1-acétate d'éthyle-pyrido[2,3-b]thiazine-2 (3H)one préparés à l'exemple 54, on récupère 3 g de 3-méthyl 1-acide acétique-pyrido[2,3-b]-thiazine-2 (3H)one sous forme de cristaux de point de fusion 189 - 190° C.

**Exemple 113**

3-méthyl 1-acide acétique-pyrido[2,3-b]-thiazine-2 (3H)-thione

Formule I: $R_1 = R_2 = R_3 = H$; $R_4 = CH_3$; X = N; Y = Z = S

On laisse sous agitation durant 24 h une solution de 3 g de 3-méthyl 1-acétate d'éthyle-pyrido[2,3-b][1,4]thiazine-2 (3H)-thione préparés à l'exemple 107 dans 25 ml de tétrahydrofuranne et 25 ml d'éthanol

contenant 0,5 g de soude dissoute dans 10 ml d'eau. Le mélange est ensuite concentré sous vide, sans chauffer, puis, après addition d'eau, les neutres sont extraits à l'éther. La phase aqueuse est acidifiée à froid par de l'acide acétique et les produits organiques extraits au chloroforme. La phase chloroformique séchée, le chloroforme évaporé, le résidu obtenu cristallise dans l'éther. Les cristaux sont essorés, lavés avec un peu d'éther et séchés. On récupère ainsi 1 g de 3-méthyl 1-acide acétique-pyrido-[2,3-b]-thiazine-2 (3H)-thione sous forme de cristaux de point de fusion 196 - 198° C.

**Exemple 114**

Chlorhydrate de 1-acide acétique-pyrido[2,3-b][1,4]pyrazine-2 (3H)-one

Formule I: $R_1 = R_2 = R_3 = R_4 = H$; $X = Y = N$; $Z = O$

On chauffe durant 7 h au reflux une solution de 7,8 g de chlorhydrate de 1-acétate d'éthyle-pyrido[2,3-b][1,4]pyrazine-2 (3H)-one préparés à l'exemple 55, dans 50 ml d'acide chlorhydrique 3N. Le mélange réactionnel est ensuite concentré sous vide et le résidu repris à l'acétone cristallise. Les cristaux essorés, lavés à l'acétone et séchés sont recristallisés dans l'acide acétique. On obtient ainsi 3,6 g du chlorhydrate de 1-acide acétique-pyrido-[2,3-b][1,4]pyrazine-2 (3H)-one sous forme de cristaux de point de fusion 260 - 265° C avec décomposition.

**Exemple 115**

6-trifluorométhyl 2H-1,4-benzothiazine-3-one 4-acide acétique

Formule I: $R_1 = 6\text{-}CF_3$; $R_2 = R_3 = R_4 = H$; $X = CH$; $Y = S = Z = O$

On porte 15 min à 60° C une solution de 10,5 g de 6-trifluorométhyl 2H-1,4-benzothiazine-3-one 4-acétate d'éthyle préparés à l'exemple 56, dans 20 ml d'éthanol contenant 2 g de soude dissoute dans 10 ml d'eau. Le mélange réactionnel est ensuite traité au charbon actif puis filtré et acidifié à froid par de l'acide chlorhydrique dilué. Les cristaux obtenus alors sont essorés, lavés à l'eau puis à l'éther isopropylique et séchés. On récupère ainsi 7 g de 6-trifluorométhyl 2H-1,4-benzothiazine-3-one 4-acide acétique sous forme de cristaux de point de fusion 165 - 167° C.

**Exemple 116**

7-fluoro 2H-1,4-benzothiazine-3-one 4-acide acétique

Formule I: $R_1 = 7\text{-}F$; $R_2 = R_3 = R_4 = H$; $X = CH$; $Y = S$; $Z = O$

Selon le mode opératoire de l'exemple 115, mais à partir de 11 g de 7-fluoro 2H-1,4,benzothiazine-3-one 4-acétate d'éthyle préparés à l'exemple 57, on récupère 7 g de 7-fluoro 2H-1,4-benzothiazine-3-one 4-acide acétique sous forme de cristaux de point de fusion 152 - 154° C.

**Exemple 117**

7-fluoro 2H-1,4-benzothiazine-3-thione 4-acide acétique

Formule I: $R_1 = 7\text{-}F$; $R_2 = R_3 = R_4 = H$; $Y = CH$: $Y = Z = S$

On abandonne 2 jours à température ambiante une solution de 8,7 g de 7-fluoro 2H-1,4-benzothiazine-3-thione 4-acétate d'éthyle, préparés à l'exemple 84, dans 100 ml de méthanol contenant 1,2 g de soude dissoute dans 15 ml d'eau. Le mélange réactionnel est ensuite concentré sous vide à froid puis dilué à l'eau qu'on extrait à l'éther. La phase aqueuse est ensuite acidifiée à froid par de l'acide chlorhydrique dilué et extraite à l'éther. Cette phase éthérée, lavée à l'eau, est séchée puis, après filtration, l'éther est évaporé sous vide. Le résidu obtenu cristallise dans un mélange éther-hexane. On récupère ainsi, après essorage et séchage des cristaux, 6 g de 7-fluoro 2H-1,4-benzothiazine-3-thione 4-acide acétique sous forme de cristaux de point de fusion 156 - 157° C.

**Exemple 118**

6-fluoro 2H-1,4-benzothiazine-3-one 4-acide acétique

Formule I: $R_1$ = 6-F; $R_2$ = $R_3$ = $R_4$ = H; X = CH; Y = S; Z = O

Selon le mode opératoire de l'exemple 115, mais à partir de 12 g de 6-fluoro 2H-1,4-benzothiazine-3-one 4-acétate d'éthyle préparés à l'exemple 58, on obtient après cristallisation dans le toluène 7,5 g de 6-fluoro 2H-1,4-benzothiazine-3-one 4-acide acétique sous forme de cristaux de point de fusion 156 - 157° C.

**Exemple 119**

6-fluoro 2H-1,4-benzothiazine-3-thione 4-acide acétique

Formule I: $R_1$ = 6-F; $R_2$ = $R_3$ = $R_4$ = H; X = CH; Y = Z = S

Selon le mode opératoire de l'exemple 117, mais à partir de 7,4 g de 6-fluoro 2H-1,4-benzothiazine-3-thione 4-acétate d'éthyle préparés à l'exemple 85, on obtient 5,3 g de 6-fluoro 2H-1,4-benzothiazine-3-thione 4-acide acétique sous forme de cristaux de point de fusion 188 - 190° C.

**Exemple 120**

8-chloro 2H-1,4-benzothiazine-3-one 4-acide acétique

Formule I: $R_1$ = 8-Cl; $R_2$ = $R_3$ = $R_4$ = H; X = CH; Y = S; Z = O

Selon le mode opératoire de l'exemple 115, mais à partir de de 14 g de 8-chloro 2H-1,4-benzothiazine-3-one 4-acétate d'éthyle préparés à l'exemple 59, on obtient 7,5 g de 8-chloro 2H-1,4-benzothiazine-3-one 4-acide acétique sous forme de cristaux de point de fusion 175 - 177° C.

**Exemple 121**

7-chloro 2H-1,4-benzothiazine-3-one 4-acide acétique

Rormule I: $R_1$ = 7-Cl; $R_2$ = $R_3$ = $R_4$ = H; X = CH; Y = S; Z = O

Selon le mode opératoire de l'exemple 115, mais à partir de 14,3 g de 7-chloro 2H-1,4-benzothiazine-3-one 4-acétate d'éthyle préparés à l'exemple 60, on récupère 8,5 g de 7-chloro 2H-1,4-benzothiazine-3-one 4-acide acétique sous forme de cristaux de point de fusion 188 - 190° C.

**Exemple 122**

6-méthoxy 2H-1,4-benzothiazine-3-one 4-acide acétique

Formule I: $R_1$ = 6-$OCH_3$; $R_2$ = $R_3$ = $R_4$ = H; X = CH; Y = S; Z = O

Selon le mode opératoire de l'exemple 115, mais à partir de 17 g de 6-méthoxy 2H-1,4-benzothiazine-3-one 4-acétate d'éthyle préparés à l'exemple 61, on récupère après recristallisation dans l'acétonitrile 8,6 g de 6-méthoxy 2H-1,4-benzothiazine-3-one 4-acide acétique sous forme de cristaux de point de fusion 152 - 155° C.

**Exemple 123**

6-trifluorométhyl 7-chloro 2H-1,4-benzothiazine-3-one 4-acide acétique

Formule I: $R_1$ = 6-$CF_3$; $R_2$ = 7-Cl; $R_3$ = $R_4$ = H; X = CH; Y = S; Z = O

Selon le mode opératoire de l'exemple 115, mais à partir de 6 g de 6-trifluorométhyl 7-chloro 2H-1,4-benzothiazine-3-one 4-acétate d'éthyle préparés à l'exemple 62, on récupère 4,3 g de 6-trifluorométhyl 7-chloro 2H-1,4-benzothiazine-3-one 4-acide acétique sous forme de cristaux de point de fusion 157 - 159° C.

**Exemple 124**

2H-1,4-benzothiazine-3-one 4-acide acétique

Formule I: $R_1 = R_2 = R_3 = R_4 = H$; $X = CH$; $Y = S$; $Z = O$

Selon le mode opératoire de l'exemple 115, mais à partir de 14 g de 2H-1,4-benzothiazine-3-one 4-acétate d'éthyle préparés à l'exemple 63, on récupère 10 g de 2H-1,4-benzothiazine-3-one 4-acide acétique sous forme de cristaux de point de fusion 152 - 154° C.

**Exemple 125**

2-méthyl 2H-1,4-benzothiazine-3-one 4-acide acétique

Formule I: $R_1 = R_2 = R_3 = H$; $R_4 = CH_3$; $X = CH$; $Y = S$; $Z = O$

Selon le mode opératoire de l'exemple 115, mais à partir de 13 g de 2-méthyl 2H-1,4-benzothiazine-3-one 4-acétate d'éthyle préparés à l'exemple 64, on obtient 8 g de 2-méthyl 2H-1,4-benzothiazine-3-one 4-acide acétique sous forme de cristaux de point de fusion 113 - 115° C.

**Exemple 126**

2-phényl 2H-1,4-benzothiazine-3-one 4-acide acétique

Formule I: $R_1 = R_2 = R_3 = H$; $R_4$ = phényl; $X = CH$; $Y = S$; $Z = O$

Selon le mode operatoire de l'exemple 115, mais à partir de 15 g de 2-phényl 2H-1,4-benzothiazine-3-one 4-acétate d'éthyle préparés à l'exemple 65, on obtient 7,8 g de 2-phényl 2H-1,4-benzothiazine-3-one 4-acide acétique sous forme de cristaux de point de fusion 155 - 157° C.

**Exemple 127**

7-chloro 2H-1,4-benzothiazine-3-thione 4-acide acétique

Formule I: $R_1$ = 7-Cl; $R_2 = R_3 = R_4 = H$; $X = CH$; $Y = Z = S$

Selon le mode opératoire de l'exemple 117, mais à partir de 20,5 g de 7-chloro 2H-1,4-benzothiazine-3-thione 4-acétate d'éthyle préparés à l'exemple 86, on obtient 8 g de 7-chloro 2H-1,4-benzothiazine-3-thione 4-acide acétique sous forme de cristaux de point de fusion 181 - 183° C.

**Exemple 128**

6-méthoxy 2H-1,4-benzothiazine-3-thione 4-acide acétique

Formule I: $R_1$ = 6-$OCH_3$; $R_2 = R_3 = R_4 = H$; $X = CH$; $Y = Z = S$

Selon le mode opératoire de l'exemple 117, mais à partir de 8 g de 6-méthoxy 2H-1,4-benzothiazine-3-thione 4-acétate d'éthyle préparés à l'exemple 87, on récupère 5,5 g de 6-méthoxy 2H-1,4-benzothiazine-3-thione 4-acide acétique sous forme de cristaux de point de fusion 163 - 165° C.

**Exemple 129**

6-trifluorométhyl 7-chloro 2H-1,4-benzothiazine-3-thione 4-acide acétique

Formule I: $R_1$ = 6-$CF_3$; $R_2$ = 7-Cl; $R_3$ = $R_4$ = H; X = H; Y = Z = S

Selon le mode opératoire de l'exemple 117, mais à partir de 5,1 g de 6-trifluorométhyl 7-chloro 2H-1,4-benzothiazine-3-thione 4-acétate d'éthyle préparés à l'exemple 88, on obtient 3,5 g de 6-trifluorométhyl 7-chloro 2H-1,4-benzothiazine-3-thione 4-acide acétique sous forme de cristaux de point de fusion 170 - 172° C.

**Exemple 130**

2-phényl 2H-1,4-benzothiazine-3-thione 4-acide acétique

Formule I: $R_1$ = $R_2$ = $R_3$ = H; $R_4$ = phényle; X = CH; Y = Z = S

Selon le mode opératoire de l'exemple 117, mais à partir de 9 g de 2-phényl 2H-1,4-benzothiazine-3-thione 4-acétate d'éthyle préparés à l'exemple 89, on obtient 4,2 g de 2-phényl 2H-1,4-benzothiazine-3-thione 4-acide acétique sous forme de cristaux de point de fusion 182 - 184° C.

**Exemple 131**

6-trifluorométhyl 2H-1,4-benzothiazine-3-thione 4-acide acétique

Formule I: $R_1$ = 6-$CF_3$; $R_2$ = $R_3$ = $R_4$ = H; X = CH; Y = Z = S

Selon le mode opératoire de l'exemple 117, mais à partir de 14 g de 6-trifluorométhyl 2H-1,4-benzothiazine-3-thione 4-acétate d'éthyle préparés à l'exemple 90, on récupère 5,5 g de 6-trifluorométhyl 2H-1,4-benzothiazine-3-thione 4-acide acétique sous forme de cristaux de point de fusion 163 - 165° C.

**Exemple 132**

2-méthyl 2H-1,4-benzothiazine-3-thione 4-acide acétique

Formule I: $R_1$ = $R_2$ = $R_3$ = H; $R_4$ = $CH_3$; X = CH; Y = Z = S

Selon le mode opératoire de l'exemple 117, mais à partir de 11 g de 2-méthyl 2H-1,4-benzothiazine-3-thione 4-acétate d'éthyle préparés à l'exemple 91, on obtient 4,5 g de 2-méthyl 2H-1,4-benzothiazine-3-thione 4-acide acétique sous forme de cristaux de point de fusion 128 - 131° C.

**Exemple 133**

2H-1,4-benzothiazine-3-thione 4-acide acétique

Formule I: $R_1$ = $R_2$ = $R_3$ = $R_4$ = H; X = CH; Y = Z = S

Selon le mode opératoire de l'exemple 117, mais à partir de 21 g de 2H-1,4-benzothiazine-3-thione 4-acétate d'éthyle préparés à l'exemple 92, on récupère 9 g de 2H-1,4-benzothiazine-3-thione 4-acide acétique sous forme de cristaux de point de fusion 187 - 188° C.

**Exemple 134**

6-chloro 2H-1,4-benzothiazine-3-one 4-acide acétique

Formule I: $R_1$ = 6-Cl; $R_2$ = $R_3$ = $R_4$ = H; X = CH; Y = S; Z = O

Selon le mode opératoire de l'exemple 115, mais à partir de 9 g de 6-chloro 2H-1,4-benzothiazine-3-one 4-acétate d'éthyle préparés à l'exemple 66, on obtient 5,5 g de 6-chloro 2H-1,4-benzothiazine-3-one 4-acide acétique sous forme de cristaux de point de fusion 151 - 153° C.

**Exemple 135**

6-chloro 2H-1,4-benzothiazine 3-thione 4-acide acétique

Formule I: $R_1$ = 6-Cl; $R_2$ = $R_3$ = $R_4$ = H; X = CH; Y = Z = S

Selon le mode opératoire de l'exemple 117, mais à partir de 11 g de 6-chloro 2H-1,4-benzothiazine-3-thione 4-acétate d'éthyle préparés à l'exemple 93, on récupère 5,2 g de 6-chloro 2H-1,4-benzothiazine-3-thione 4-acide acétique sous forme de cristaux de point de fusion 186 - 188° C.

**Exemple 136**

6-fluoro 2-méthyl 2H-1,4-benzothiazine-3-one 4-acétique acide

Formule I: $R_1$ = 6-F; $R_3$ = $CH_3$; $R_2$ = $R_4$ = H; X = CH; Y = S; Z = O

Selon le mode opératoire de l'exemple 115, mais à partir de 10 g de 6-fluoro 2-méthyl 2H-1,4-benzothiazine-3-one 4-acétate d'éthyle péparés à l'exemple 67, on récupère 6,5 g de 6-fluoro 2-méthyl 2H-1,4-benzothiazine-3-one 4-acétique acide sous forme de cristaux de point de fusion 139 - 141° C.

**Exemple 137**

6-fluoro 2-méthyl 2H-1,4-benzothiazine-3-thione 4-acétique acide

Formule I: $R_1$ = 6-F; $R_3$ =$CH_3$; $R_2$ = $R_4$ = H; X = CH; Y = Z =S

Selon le mode opératoire del'exemple 117, mais à partir de 6 g de 6-fluoro-2-méthyl 2H-1,4-benzothiazine-3-thione 4-acétate d'éthyle préparés à l'exemple 94, on obtient 3 g de 6-fluoro 2-méthyl 2H-1,4-benzothiazine-3-thione 4-acétique acide sous forme de cristaux de point de fusion 138 - 140° C.

**Exemple 138**

6-chloro 2-méthyl 2H-1,4-benzothiazine-3-one 4-acétique acide

Formule I: $R_1$ = 6-Cl; $R_3$ = $CH_3$; $R_2$ = $R_4$ = H; X = CH; Y = S; Z = O

Selon le mode opératoire de l'exemple 115, mais à partir de 7 g de 6-chloro 2-méthyl 2H-1,4-benzothiazine-3-one 4-acétate d'éthyle préparés à l'exemple 68, on obtient 4,9 g de 6-chloro 2-méthyl 2H-1,4-benzothiazine-3-one 4-acétique acide sous forme de cristaux de point de fusion 129 - 131° C.

**Exemple 139**

6-chloro 2-phényl 2H-1,4-benzothiazine-3-one 4-acétique acide

Formule: $R_1$ = 6-Cl; $R_3$ = phényle; $R_2$ = $R_4$ = H; X = CH; Y = S; Z = O

Selon le mode opératoire de l'exemple 115, mais à partir de 8 g de 6-chloro 2-phényl 2H-1,4-benzothiazine-3-one 4-acétate d'éthyle préparés à l'exemple 69, on récupère 5,3 g de 6-chloro 2-phényl 2H-1,4-benzothiazine-3-one 4-acétique acide sous forme de cristaux de point de fusion 169 - 170° C.

**Exemple 140**

6-chloro 2-méthyl 2H-1,4-benzothiazine-3-thione 4-acétique acide

Formule I: $R_1$ = 6-Cl; $R_3$ = $CH_3$; $R_2$ = $R_4$ = H; X = CH; Y = Z = S

Selon le mode opératoire de l'exemple 117, mais à partir de 9 g de 6-chloro 2-méthyl 2H-1,4-benzothiazine-3-thione 4-acétate d'éthyle préparés à l'exemple 95, on récupère 4,5 g de 6-chloro 2-méthyl 2H-1,4-benzothiazine-3-thione 4-acétique acide sous forme de cristaux de point de fusion 126 - 128° C.

**Exemple 141**

2,2-diméthyl 2H-1,4-benzothiazine-3-one 4-acide acétique

Formule I: $R_1$ = $R_2$ = H; $R_3$ = $R_4$ = $CH_3$; X = CH; Y = S; Z = O

Selon le mode opératoire de l'exemple 115, mais à partir de 15 g de 2,2-dimèthyl 2H-1,4-benzothiazine-3-one 4-acétate d'éthyle préparés à l'exemple 70, on obtient 12 g de 2,2-diméthyl 2H-1,4-benzothiazine-3-one 4-acide acétique sous forme de cristaux de point de fusion 114 - 116° C.

**Exemple 142**

6-chloro 2-phényl 2H-1,4-benzothiazine-3-thione 4-acide acétique

Formule I: $R_1$ = 6-Cl; $R_3$ = phényle; $R_2$ = $R_4$ = H; X = CH; Y = Z = S

Selon le mode opératoire de l'exemple 117, mais en utilisant 11 g de 6-chloro 2-phényl 2H-1,4-benzothiazine-3-thione 4-acétate d'éthyle préparés à l'exemple 96, on récupère 4,5 g de 6-chloro 2-phényl 2H-1,4-benzothiazine-3-thione 4-acide acétique sous forme de cristaux de point de fusion 199 - 201° C.

**Exemple 143**

2-para-chlorophényl 2H-1,4-benzothiazine-3-one 4-acide acétique

Formule I: $R_1$ = $R_2$ = $R_3$ = H; $R_4$ =para-chlorophényle; X = CH; Y = S; Z = O

Selon le mode opératoire de l'exemple 115, mais à partir de 7 g de 2-para-chlorophényl 2H-1,4-benzothiazine-3-one 4-acétate d'éthyle préparés à l'exemple 71, on récupère 5 g de 2-para-chlorophényl 2H-1,4-benzothiazine-3-one 4-acide acétique sous formé de cristaux de point de fusion 138 - 140° C.

**Exemple 144**

2-ortho-chlorophényl 2H-1,4-benzothiazine-3-one 4-acide acétique

Formule I: $R_1$ = $R_2$ = $R_3$ = H; $R_4$ = ortho-chlorophényl; X = CH; Y = S; Z = O

Selon le mode opératoire de l'exemple 115, mais à partir de 10 g de 2-orthochlorophényl 2H-1 -benzothiazine-3-one 4-acétate d'éthyle préparés à l'exemple 72, on obtient 6,3 g de 2-ortho-chlorophényl 2H-1,4-benzothiazine-3-one 4-acide acétique sous forme de cristaux de point de fusion 164 - 165° C.

**Exemple 145**

7-chloro 2-méthyl 2H-1,4-benzothiazine-3-one 4-acide acétique

Formule I: $R_1$ = 7-Cl; $R_3$ = $CH_3$; $R_2$ = $R_4$ = H; X = CH; Y = S; Z = O

Selon le mode opératoire de l'exemple 115, mais à partir de 7,2 g de 7-chloro 2-méthyl 2H-1,4-benzothiazine-3-one 4-acétate d'éthyle, préparés à l'exemple 83, on récupère 3,2 g de 7-chloro 2-méthyl 2H-1,4-benzothiazine-3-one 4-acide acétique sous forme de cristaux de point de fusion 117 - 119°C.

**Exemple 146**

2,2-diméthyl 2H-1,4-benzothiazine-3-thione 4-acide acétique

Formule I: $R_1 = R_2 = H$; $R_3 = R_4 = CH_3$; X = CH; Y = Z = S

Selon le mode opératoire de l'exemple 117, mais à partir de 11,2 g de 2,2-diméthyl 2H-1,4-benzothiazine-3-thione 4-acétate d'éthyle préparés à l'exemple 97, on obtient 5,8 g de 2,2-diméthyl 2H-1,4-benzothiazine-3-thione 4-acide acétique sous forme de cristaux de point de fusion 176 - 178°C.

**Exemple 147**

2-para-chlorophényl 2H-1,4-benzothiazine-3-thione 4-acide acétique

Formule I: $R_1 = R_2 = R_3 = H$; $R_4$ = para-chlorophényle; X = CH; Y = Z = S

Selon le mode opératoire de l'exemple 117, mais à partir de 11,2 g de 2-para-chlorophényl 2H-1,4-benzothiazine-3-thione 4-acétate d'éthyle préparés à l'exemple 98, on obtient 5,9 g de 2-para-chlorophényl 2H-1,4-benzothiazine-3-thione 4-acide acétique sous forme de cristaux de point de fusion 173 - 174°C.

**Exemple 148**

2-ortho-chlorophényl 2H-1,4-benzothiazine-3-thione 4-acide acétique

Formule I: $R_1 = R_2 = R_3 = H$; $R_4$ = ortho-chlorophényl; X = CH; Y = Z = S

Selon le mode opératoire de l'exemple 117, mais à partir de 18 g de 2-orthochlorophényl 2H-1,4-benzothiazine-3-thione 4-acétate d'éthyle synthétisés à l'exemple 101, on récupère 8,2 g de 2-ortho-chlorophényl 2H-1,4-benzothiazine-3-thione 4-acide acétique sous forme de cristaux de point de fusion 179 - 181°C.

**Exemple 149**

6,7-dichloro 2H-1,4-benzothiazine-3-thione 4-acide acétique

Formule I: $R_1$ = 6-Cl; $R_2$ = 7-Cl; $R_3 = R_4 = H = H$; X = CH; Y = Z = S

Selon le mode opératoire de l'exemple 117, mais à partir de 2,4 g de 6,7-dichloro 2H-1,4-benzothiazine-3-thione 4-acétate d'éthyle préparés à l'exemple 99, on obtient 1 g de 6,7-dichloro 2H-1,4-benzothiazine-3-thione 4-acide acétique sous forme de cristaux de point de fusion 185 - 187°C.

**Exemple 150**

7-chloro 2-méthyl 2H-1,4-benzothiazine-3-thione 4-acide acétique

Formule I: $R_1$ = 7-Cl; $R_3 = CH_3$; $R_2 = R_4 = H$; X = CH; Y = Z = S

Selon le mode opératoire de l'exemple 117, mais à partir de 9,2 g de 7-chloro 2-méthyl 2H-1,4-benzothiazine-3-thione 4-acétate d'éthyle préparés à l'exemple 100, on obtient 5 g de 7-chloro 2-méthyl 2H-1,4-benzothiazine-3-thione 4-acide acétique sous forme de cristaux de point de fusion 136 - 138°C.

**Exemple 151**

2-méthyl 2-(2'-pyridyl) 2H-1,4-benzothiazine-3-one 4-acide acétique

Formule I: $R_1 = R_2 = H$; $R_3 = CH_3$; $R_4$ = 2'-pyridyl; X = CH; Y = S; Z = O

Selon le mode opératoire de l'exemple 115, mais à partir de 12 g de 2-méthyl 2-(2'-pyridyl)2H-1,4-benzothiazine-3-one 4-acétate d'éthyle préparés à l'exemple 74 et en neutralisant à la fin de la saponification par l'acide acétique au lieu de l'acide chlorhydrique, on obtient 5,6 g de 2-méthyl 2-(2'-pyridyl) 2H-1,4-benzothiazine-3-one 4-acide acétique sous forme de cristaux de point de fusion 194 - 196° C.

**Exemple 152**

6-fluoro 2H-1,4-benzoxazine-3-thione 4-acide acétique

Formule I: $R_1$ = 6-F; $R_2 = R_3 = R_4 = H$; X = CH; Y = O; Z = S

Selon le mode opératoire de l'exemple 117, mais en utilisant 8,8 g de 6-fluoro 2H-1,4-benzoxazine-3-thione 4-acétate d'éthyle préparés à l'exemple 102, on obtient après filtration sur gel de silice en éluant au benzène, 1,5 g de 6-fluoro 2H-1,4-benzoxazine-3-thione 4-acide acétique sous forme de cristaux de point de fusion 153 - 155° C.

**Exemple 153**

6-fluoro 3,4-dihydroquinoline 2-thione 1-acide acétique

Formule I: $R_1$ = 6-F; $R_2 = R_3 = R_4 = H$; X = CH; Y = $CH_2$; Z = S

Selon le mode opératoire de l'exemple 117, mais en utilisant 6,5 g de 6-fluoro 3,4-dihydroquinoline 2-thione 1-acétate d'éthyle préparés à l'exemple 103, on obtient après recristallisation dans l'acétonitrile 2,9 g de 6-fluoro 3,4-dihydroquinoline 2-thione 1-acide acétique sous forme de cristaux de point de fusion 177 - 180° C.

**Exemple 154**

6-chloro 3,4-dihydroquinoline 2-thione 1-acide acétique

Formule I: $R_1$ = 6-Cl; $R_2 = R_3 = R_4 = H$; X = CH; Y = $CH_2$; Z = S

Selon le mode opératoire de l'exemple 117, mais en utilisant 9,4 g de 6-chloro 3,4-dihydroquinoline 2-thione 1-acétate d'éthyle préparés à l'exemple 104, on récupère 3,5 g de 6-chloro 3,4-dihydroquinoline 2-thione 1-acide acétique sous forme de cristaux de point de fusion 165° C.

**Exemple 155**

7-fluoro 3,4-dihydroquinoline 2-thione 1-acide acétique

Formule I: $R_1$ = 7-F; $R_2 = R_3 = R_4 = H$; X = CH; Y = $CH_2$; Z = S

Selon le mode opératoire de l'exemple 117, mais en utilisant 15,7 g de 7-fluoro 3,4-dihydroquinoline 2-thione 1-acétate d'éthyle préparés à l'exemple 105, on récupère 5,8 g de 7-fluoro 3,4-dihydroquinoline 2-thione 1-acide acétique sous forme de cristaux de point de fusion 154 - 156° C.

**Exemple 156**

7-chloro 3,4-dihydroquinoline 2-thione 1-acide acétique

Formule I: $R_1$ = 7-Cl; $R_2$ = $R_3$ = $R_4$ = H; X = CH; Y = $CH_2$ = Z = S

Selon le mode opératoire de l'exemple 117, mais en partant de 12 g de 7-chloro 3,4-dihydroquinoline 2-thione 1-acétate d'éthyle préparés à l'exemple 106, on récupère après recristallisation dans un mélange toluène-acétonitrile 4 g de 7-chloro 3,4-dihydroquinoline 2-thione 1-acide acétique sous forme de cristaux de point de fusion 148 - 155° C.

**Exemple 157**

2-para-fluorophényl 2H-1,4-benzothiazine-3-one 4-acide acétique

Formule I: $R_1$ = $R_2$ = $R_3$ = H; $R_4$ = para-fluorophényle; X = CH; Y = S; Z = O

Selon le mode opératoire de l'exemple 115, mais en partant de 12 g de 2-para-fluorophényl 2H-1,4-benzothiazine-3-one 4-acétate d'éthyle préparés à l'exemple 80, on récupère 7 g de 2-para-fluorophényl 2H-1,4-benzothiazine-3-one 4-acide acétique sous forme de cristaux de point de fusion 140 - 141° C.

**Exemple 158**

2-ortho-fluorophényl 2H-1,4-benzothiazine-3-one 4-acide acétique

Formule I: $R_1$ = $R_2$ = $R_3$ = H; $R_4$ = ortho-fluorophényl; X = CH; Y = S; Z = O

Selon le mode opératoire de l'exemple 115, mais en partant de 12 g de 2-ortho-fluorophényl 2H-1,4-benzothiazine-3-one 4-acétate d'éthyle préparés à l'exemple 81, on obtient 10,2 g de 2-ortho-fluorophényl 2H-1,4-benzothiazine-3-one 4-acide acétique sous forme de cristaux de point de fusion 162 - 163° C.

**Exemple 159**

2-para-fluorophényl 2H-1,4-benzothiazine-3-thione 4-acide acétique

Formule I: $R_1$ = $R_2$ = $R_3$ = H; $R_4$ = para-fluorophényl; X = CH; Y = Z = S

Selon le mode opératoire de l'exemple 117, mais à partir de 12 g de 2-para-fluorophényl 2H-1,4-benzothiazine-3-thione 4-acétate d'éthyle préparés à l'exemple 108, on obtient après cristallisation dans un mélange d'éther isopropylique et de pentane 4,2 g de 2-para-fluorophényl 2H-1,4-benzothiazine-3-thione 4-acide acétique sous forme de cristaux de point de fusion 160 - 161° C.

**Exemple 160**

2-ortho-fluorophényl 2H-1,4-benzothiazine-3-thione 4-acide acétique

Formule I: $R_1$ = $R_2$ = $R_3$ = H; $R_4$ = ortho-fluorophényle; X = CH; Y = Z = S

Selon le mode opératoire de l'exemple 117, mais à partir de 19 g de 2-ortho-fluorophényl 2H-1,4-benzothiazine-3-thione 4-acétate d'éthyle obtenus à l'exemple 109, on récupère après cristallisation dans un mélange d'éther isopropylique et de pentane 7 g de 2-ortho-fluorophényl 2H-1,4-benzothiazine-3-thione 4-acide acétique sous forme de cristaux de point de fusion 180 - 182° C.

**Exemple 161**

4-fluoro 2-nitrophénylthio α,α'-diméthyl acétique acide

Formule VIII: $R_1$ = 4-F; $R_3$ = $R_4$ = $CH_3$; $R_2$ = H; X = CH; Y = S; R'' = H

Selon le mode opératoire de l'exemple 1, mais en utilisant 48 g de 2,5-difluoronitrobenzène et 36,2 g d'α-mercaptoisobutyrique acide, on obtient après acidification à l'acide acétique, extraction à l'éther et cristallisation du résidu dans un mélange éther isopropylique et pentane 37,4 g de 4-fluoro 2-nitrophénylthio α,α'-diméthyl acétique acide sous forme de cristaux de point de fusion 104°C.

**Exemple 162**

4-fluoro 2-nitrophénylthio α,α'-diméthyl acétate d'éthyle

Formule VIII: $R_1$ = 4-F; $R_3$ = $R_4$ = $CH_3$; $R_2$ = H; X = CH; Y = S; R'' = $C_2H_5$

Selon le mode opératoire de l'exemple 19, mais à partir de 37,4 g de 4-fluoro 2-nitrophénylthio α,α'-diméthyl acétique acide, préparés à l'exemple 161, on obtient 38,5 g de 4-fluoro 2-nitrophénylthio α,α'-diméthyl acétate d'éthyle sous forme d'une huile utilisée brute pour l'étape suivante.

**Exemple 163**

6-fluoro 2,2-diméthyl 2H-1,4-benzothiazine-3 (4H)-one

Formule III: $R_1$ = 6-F; $R_3$ = $R_4$ = $CH_3$; $R_2$ = H; X = CH; Y = S

Selon le mode opératoire de l'exemple 28, mais en utilisant 38,5 g de 4-fluoro 2-nitrophénylthio α,α'-diméthyl acétate d'éthyle, préparés à l'exemple 162, on obtient 18,5 g de 6-fluoro 2,2-diméthyl 2H-1,4-benzothiazine-3 (4H)-one sous forme de cristaux de point de fusion 176°C.

**Exemple 164**

6-fluoro 2,2-diméthyl 2H-1,4-benzothiazine-3-one 4-acétate d'éthyle

Formule II: $R_1$ = 6-F; $R_3$ = $R_4$ = $CH_3$; $R_2$ = H; X = CH; Y = S; Z = O; R' = $C_2H_5$

Selon le mode opératoire de l'exemple 52, mais à partir de 18,5 g de 6-fluoro 2,2-diméthyl 2H-1,4-benzothiazine-3 (4H)-one préparés à l'exemple 163, on obtient 25 g de 6-fluoro 2,2-diméthyl 2H-1,4-benzothiazine-3-one 4-acétate d'éthyle sous forme de cristaux de point de fusion 78°C.

**Exemple 165**

6-fluoro 2,2-diméthyl 2H-1,4-benzothiazine-3-thione 4-acétate d'éthyle

Formule II: $R_1$ = 6-F; $R_3$ = $R_4$ = $CH_3$; $R_2$ = H; X = CH; Y = Z = S; R' = $C_2H_5$

Selon le mode opératoire de l'exemple 84, mais à partir de 18 g de 6-fluoro-2,2-diméthyl 2H-1,4-benzothiazine-3-one 4-acétate d'éthyle, préparés à l'exemple 164, on obtient après filtration sur gel de silice en éluant au toluène et après cristallisation dans le pentane 8 g de 6-fluoro 2,2-diméthyl 2H-1,4-benzothiazine-3-thione 4-acétate d'éthyle sous forme de cristaux de point de fusion 60 - 62°C.

**Exemple 166**

6-fluoro 2,2-diméthyl 2H-1,4-benzothiazine-3-one 4-acide acétique

Formule I: $R_1$ = 6-F; $R_3$ = $R_4$ = $CH_3$; $R_2$ = H; X = CH; Y = S; Z = O

Selon le mode opératoire de l'exemple 115, mais à partir de 7 g de 6-fluoro 2,2-diméthyl 2H-1,4-benzothiazine-3-one 4-acétate d'éthyle, exemple 164, on obtient après recristallisation dans le tétrachlorure de carbone 4 g de 6-fluoro 2,2-diméthyl 2H-1,4-benzothiazine-3-one 4-acide acétique sous forme de cristaux de point de fusion 137 - 138° C.

**Exemple 167**

6-fluoro 2,2-diméthyl 2H-1,4-benzothiazine-3-thione 4-acide acétique

Formule I: $R_1$ = 6-F; $R_3$ = $R_4$ = $CH_3$; $R_2$ = H; X = CH; Y = Z = S

Selon le mode opératoire de l'exemple 117, mais à partir de 7,8 g de 6-fluoro 2,2-diméthyl 2H-1,4-benzothiazine-3-thione 4-acétate d'éthyle préparé à l'exemple 165, on récupère 5,8 g de 6-fluoro 2,2-diméthyl 2H-1,4-benzothiazine-3-thione 4-acide acétique sous forme de cristaux de point de fusion 171-172° C.

**Exemple 168**

(Préparation d'un produit selon l'invention par hydrolyse en utilisant le bicarbonate de potassium)

2-ortho-fluorophényl 2H-1,4-benzothiazine-3-thione 4-acide acétique
code 5180-48

Formule I: $R_1$ = $R_2$ = $R_3$ = H; $R_4$ = orthofluorophényle; X = CH; Y = Z = S

On porte au reflux durant 3 h une solution de 5,6 g de 2-orthofluorophényl 2H-1,4-benzothiazine-3-thione 4-acétate d'éthyle obtenus à l'exemple 109 et de 1,6 g de bicarbonate de potassium dans 100 ml d'éthanol et 50 ml d'eau. La solution est ensuite refroidie, additionnée d'eau et extraite à l'éther. La phase aqueuse est ensuite acidifiée à froid par de l'acide chlorhydrique 0,1 N et extraite à l'éther; cet éther séché sur sulfate de sodium est évaporé sous vide. Le résidu obtenu cristallise, il est repris par du pentane et essoré. On récupère ainsi 3,9 g de 2-orthofluorophényl 2H-1,4-benzothiazine-3-thione 4-acide acétique sous forme de cristaux de point de fusion 180 - 182° C et identiques à ceux obtenus à l'exemple 160.

**Pharmacologie**

Principe

L'activité inhibitrice de l'aldose réducatase est écaluée in vitro à partir d'un homogénat de cristallin de rat utilisé comme source d'enzyme. Le substrat utilisé est le DL-glycéraldéhyde qui est transformé par l'aldose réductase en glycérol, en présence de NADPH (*). Cette réaction est suivie par spectrophotométrie à 340 nm, en l'absence et en présence des inhibiteurs à tester, la variation de densité optique étant proportionnelle à l'oxydation du coenzyme réduit.

Résultats

Les résultats figurent dans le tableau I ci-dessous et représentent pour différents exemples le pourcentage d'inhibition de l'activité enzymatique par rapport à l'activité témoin en fonction des différentes concentrations (M.I-1) utilisées. Pour les dérivés les plus actifs, la concentration inhibitrice 50 a été déterminée.

**Tableau I**

| Concentration (mole/litre) | Inhibition par rapport à l'activité témoin, % $10^{-4}$ | $10^{-5}$ | $10^{-6}$ | $10^{-7}$ | $10^{-8}$ | $10^{-9}$ | CI50 nm/l (**) |
|---|---|---|---|---|---|---|---|
| Exemple 110 | 95 | 82 | 37 | 11 | 4 | 9 | |
| Exemple 111 | 96 | 80 | | 5 | | | |
| Exemple 114 | 95 | 86 | 6 | | | | |
| Exemple 115 | 90 | 57 | | 3 | | | |
| Exemple 116 | 100 | 95 | 25 | | | | |
| Exemple 117 | 100 | 100 | | 65 | 5 | | 82 ± 1 |
| Exemple 118 | 98 | 91 | 10 | | | | |
| Exemple 119 | 100 | 98 | | 67 | 22 | | 46 ± 2 |
| Exemple 121 | 97 | 92 | | 16 | | | |
| Exemple 123 | 97 | 81 | | 0 | | | |
| Exemple 124 | 97 | 86 | | 10 | | | |
| Exemple 125 | 99 | 91 | | 12 | | | |
| Exemple 127 | | 98 | | 93 | 28 | | |
| Exemple 129 | | 74 | | 25 | 5 | | |
| Exemple 130 | | 99 | | 95 | 17 | | |
| Exemple 132 | | 95 | | 92 | 16 | | 42 ± 13(***) |
| Exemple 133 | | 98 | | 69 | 18 | | 54 ± 5 |
| Exemple 136 | | 95 | | 30 | 11 | | |
| Exemple 137 | | 94 | | 80 | 13 | | |
| Exemple 151 | 96 | 73 | | 16 | | | |
| Exemple 152 | 100 | 99 | | 54 | | | 86 ± 4 |
| Exemple 154 | | 94 | | 56 | 29 | | 91 ± 15 |

(*) Nicotinamide Adénine Dinucléotide PHosphate forme réduite
(**) n = moyenne de 3 déterminations
(***) n = moyenne de 2 déterminations

En thérapeutique humaine, les composés de formule I et éventuellement leurs sels d'addition non toxiques peuvent être administrés, notamment par voie orale, sous forme de gélules ou comprimés renfermant de 50 à 300 mg de principe actif.

Ces différents composés de formule I ou leurs sels d'addition non toxiques présentent une activité inhibitrice de l'aldose réductase. Ils peuvent donc être administrés avec profit pour traiter certaines complications de la maladie diabétique (cataracte et neuropathies périphériques).

Les produits des différents exemples décrits sont peu toxiques dans la mesure où les doses létales 50, déterminées chez le rat par voie orale, sont toutes supérieures à 300 mg.$kg^{-1}$

**Tableau II**

Exemple 110

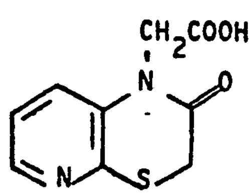

Code 5155-01

Exemple 111

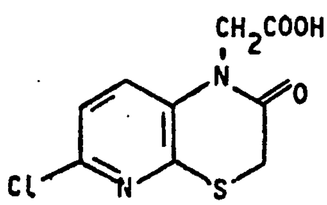

Code 5155-02

Exemple 112

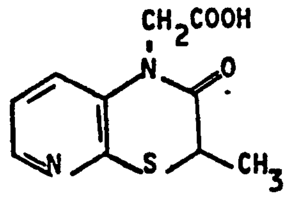

Code 5155-06

Exemple 113

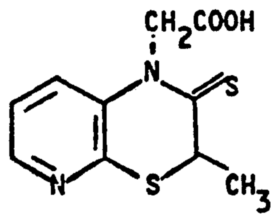

Code 5155-07

Exemple 114

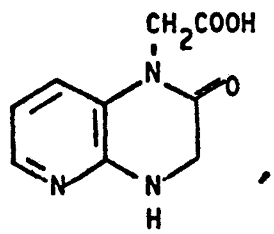

, HCl

Code 5155-03

Exemple 115

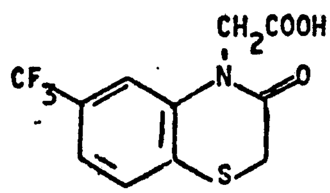

Code 5180-01

Exemple 116

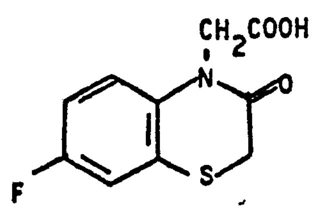

Code 5180-04

Exemple 117

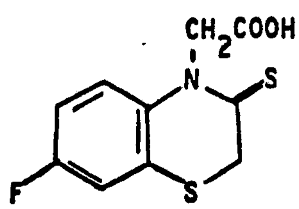

Code 5180-09

Exemple 118

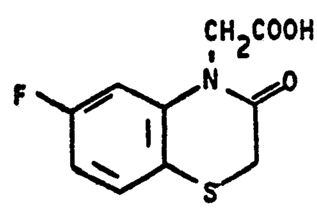

Code 5180-10

Exemple 119

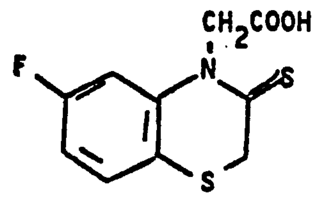

Code 5180-11

Exemple 120

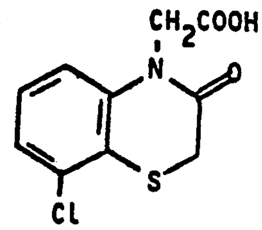

Code 5180-14

Exemple 121

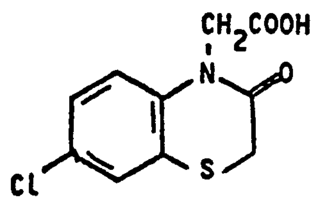

Code 5180-15

Exemple 122

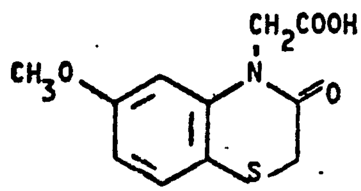

Code 5180-16

Exemple 123

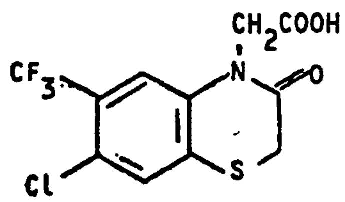

Code 5180-17

Exemple 124

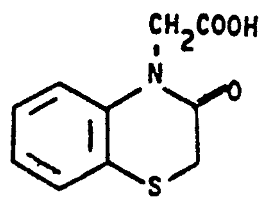

Code 5180-18

Exemple 125

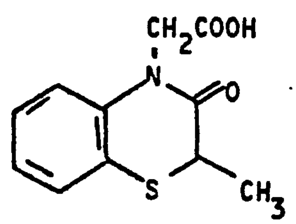

Code 5180-19

Exemple 126

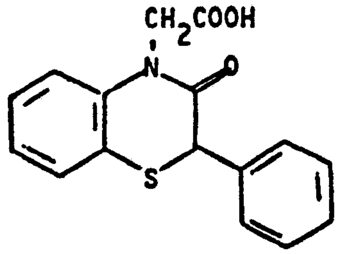

Code 5180-20

Exemple 127

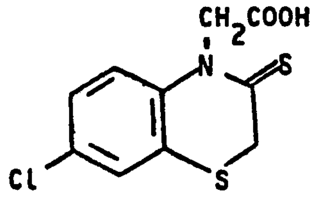

Code 5180-21

Exemple 128

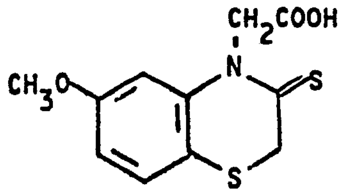

Code 5180-22

Exemple 129

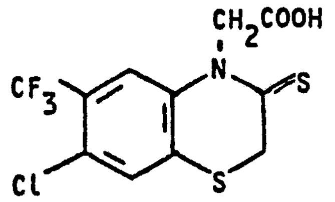

Code 5180-23

Exemple 130

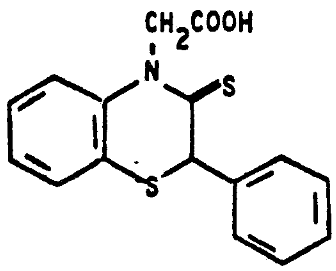

Code 5180-24

Exemple 131

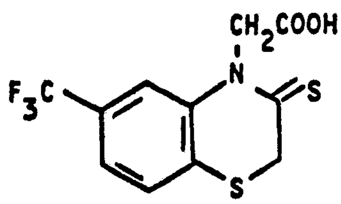

Code 5180-25

Exemple 132

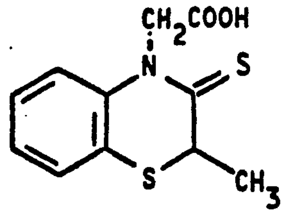

Code 5180-26

Exemple 133

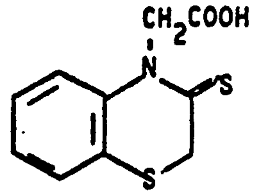

Code 5180-27

Exemple 134

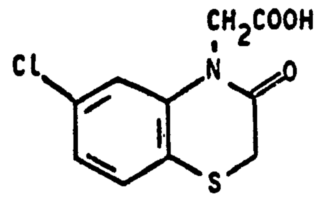

Code 5180-28

Exemple 135

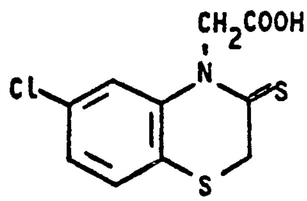

Code 5180-29

Exemple 136

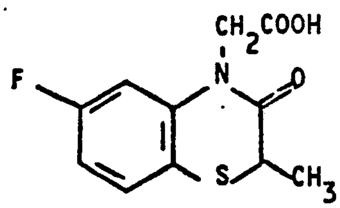

Code 5180-30

Exemple 137

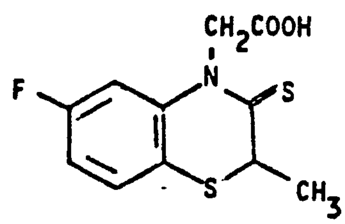

Code 5180-31

Exemple 138

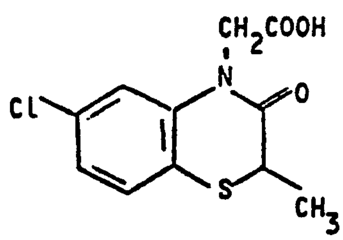

Code 5180-32

Exemple 139

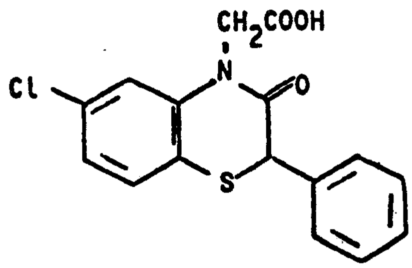

Code 5180-33

Exemple 140

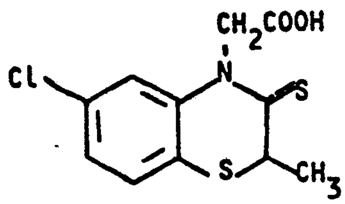

Code 5180-34

Exemple 141

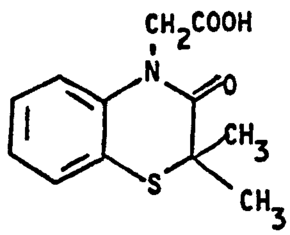

Code 5180-35

Exemple 142

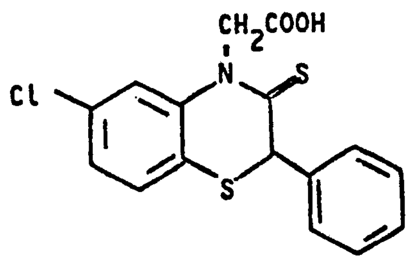

Code 5180-36

Exemple 143

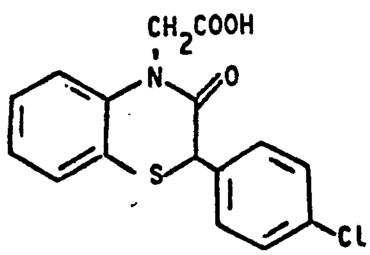

Code 5180-37

Exemple 144

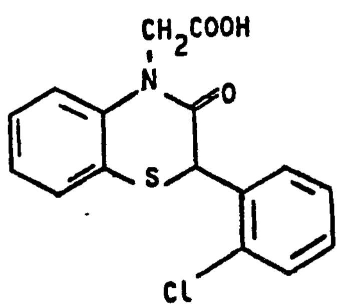

Code 5180-38

Exemple 145

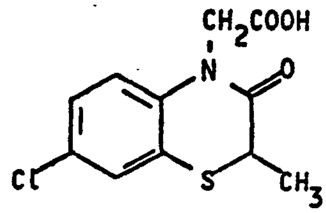

Code 5180-39

Exemple 146

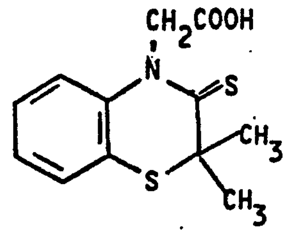

Code 5180-40

Exemple 147

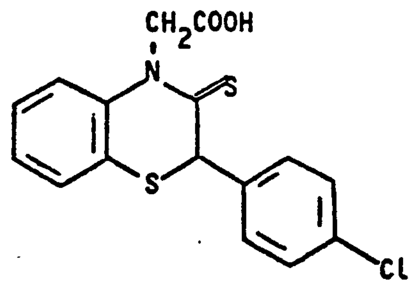

Code 5180-41

Exemple 149

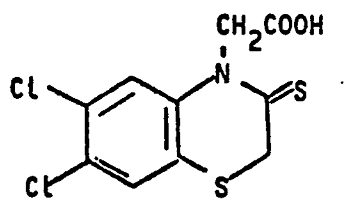

Code 5180-42

Exemple 150

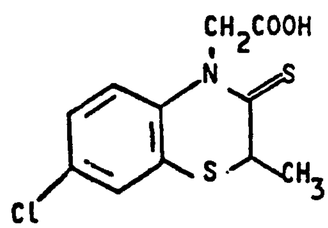

Code 5180-43

Exemple 152

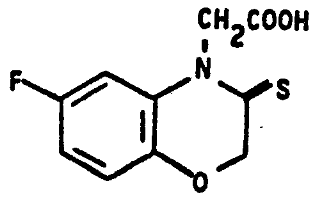

Code 5180-13

Exemple 153

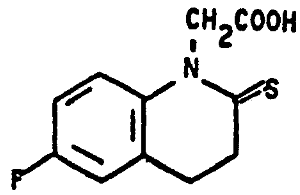

Code 5204-04

Exemple 154

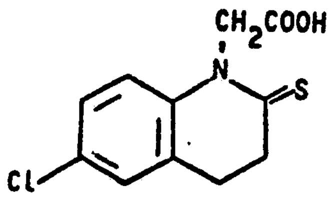

Code 5204-05

Exemple 155

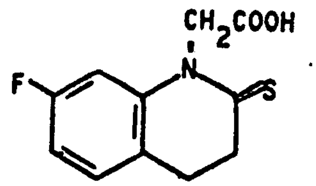

Code 5204-07

Exemple 156

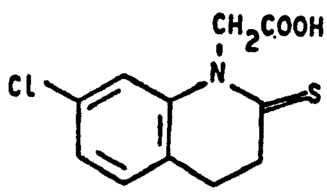

Code 5204-08

Exemple 166

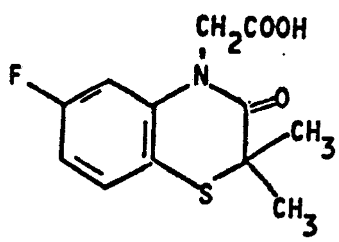

Code 5180-49

Exemple 167

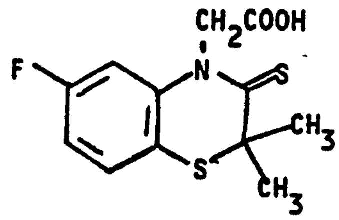

Code 5180-50

Exemple 148

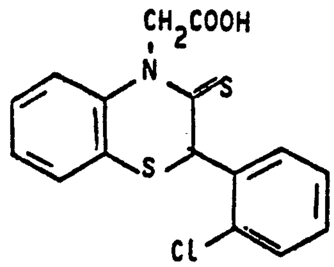

Code 5180-44

Exemple 157

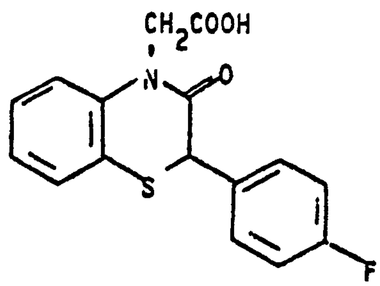

Code 5180-45

Exemple 158

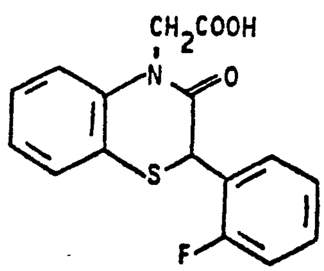

Code 5180-46

Exemple 159

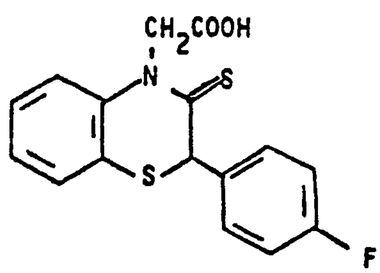

Code 5180-47

Exemple 160

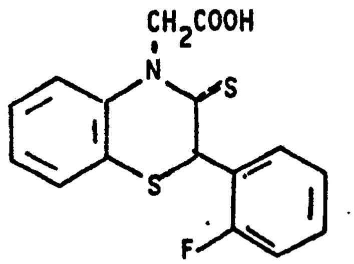

Code 5180-48

Exemple 151

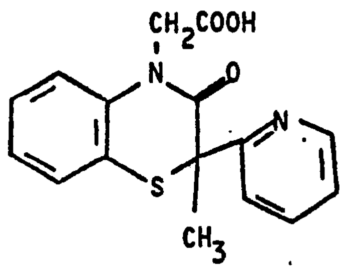

Code 5177-01

**Revendications** pour les états contractants: BE, CH, DE, FR, GB, IT, LI, LU, NL, SE

1. Nouveaux composés, caractérisés en ce qu'ils répondent à la formule:

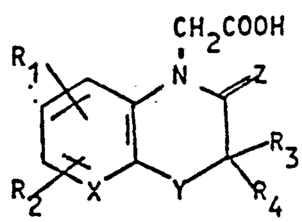

(I)

dans laquelle

| | |
|---|---|
| Z | représente l'atome de soufre, mais peut représenter également l'oxygène quand Y représente le soufre ou quand X = N et Y = NH; |
| Y | représente l'atome d'oxygène ou l'atome de soufre ou un méthylène; |
| Y | peut en outre représenter NH quand X est l'azote; |
| X | représente CH ou l'atome d'azote; |
| $R_1$ et $R_2$ | peuvent représenter l'hydrogène, un halogène, un groupement trifluorométhyle, méthoxy, thiométhyle, thiotrifluorométhyle, trifluorométhoxy; |
| $R_3$ et $R_4$ | peuvent représenter l'hydrogène, un alkyle inférieur en $C_1$-$C_5$ ramifié ou non ramifié, un noyau phényle ou pyridyle et leurs sels non toxiques d'addition. |

2. Nouveaux composés selon la revendication 1, caractérisés en ce que Z représente le soufre.

3. Nouveaux composés selon la revendication 1 ou 2, caractérisés en ce que Y représente le soufre.

4. Nouveaux composés selon l'une quelconque des revendications 1 à 3, caractérisés en ce que $R_1$ = H et $R_2$ représente le fluor.

5. Nouveaux composés selon l'une quelconque des revendications 1 à 4, caractérisés en ce que $R_3$ = H et $R_4$ représente un méthyle ou un phényle.

6. Nouveau composé selon la revendication 1, caractérisé en ce qu'il répond à la formule:

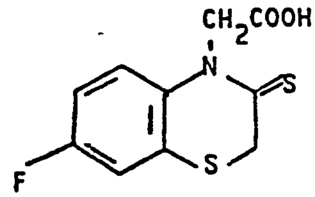

7. Nouveau composé selon la revendication 1, caractérisé en ce qu'il répond à la formule:

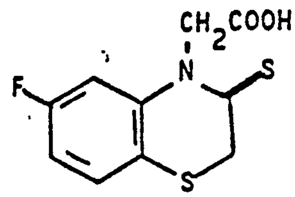

8. Nouveau composé selon la revendication 1, caractérisé en ce qu'il répond à la formule:

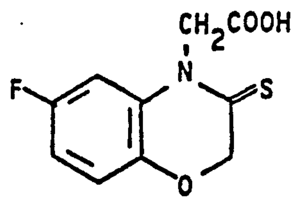

9. Nouveau composé selon la revendication 1, caractérisé en ce qu'il répond à la formule:

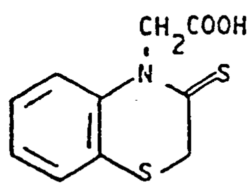

ou

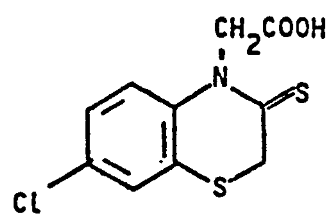

10. Nouveau composé selon la revendication 1, caractérisé en ce qu'il répond à la formule:

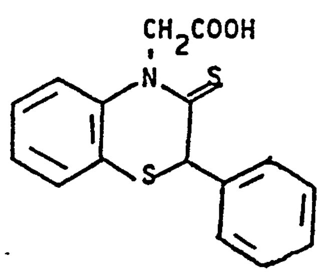

11. Nouveau composé selon la revendication 1, caractérisé en ce qu'il répond à la formule:

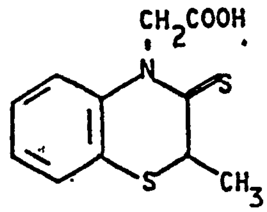

12. Nouveaux composés, caractérisés en ce qu'ils sont choisis parmi les composés suivants:

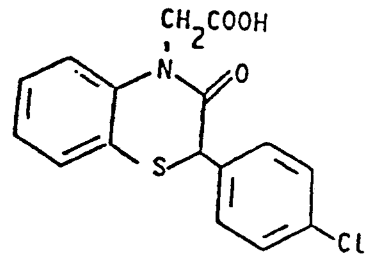

Code 5180-37

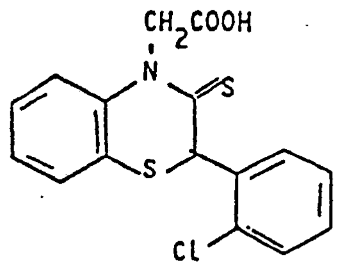

Code 5180-44

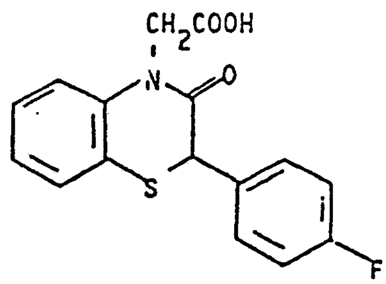

Code 5180-45

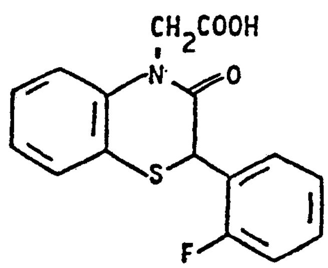

Code 5180-46

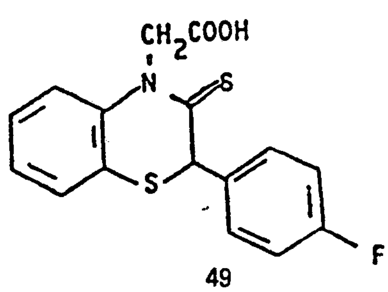

Code 5180-47

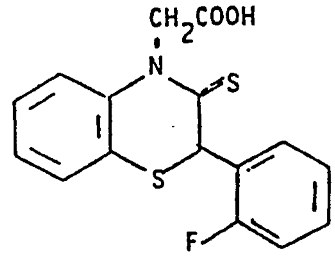

Code 5180-48

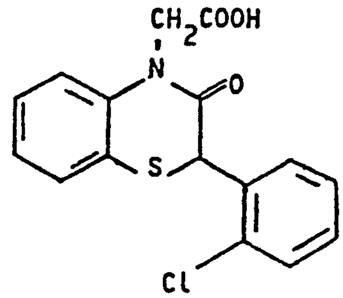

Code 5180-38

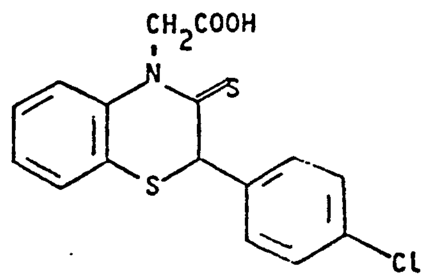

Code 5180-41

13. Nouveau composé selon la revendication 1, caractérisé en ce que Z = S, Y = S, X = CH, $R_2$ = $R_3$ = H, $R_1$ = H ou halogène, et $R_4$ = H, méthyle ou phényle.

14. Nouveau composé selon la revendication 1 ou 3, caractérisé en ce qu'il répond à la formule:

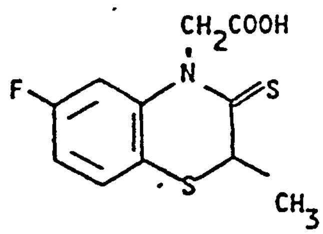

15. Procédé de préparation des nouveaux composés selon l'une quelconque des revendications 1 à 14, caractérisé en ce que l'on réalise l'hydrolyse en milieu basique ou acide d'esters de formule:

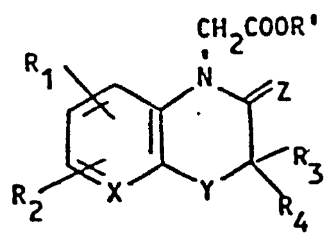

(II)

où $R_1$, $R_2$, $R_3$, $R_4$, X, Y et Z sont tels que définis dans l'une quelconque des revendications 1 à 14 et R' représente un radical alkyle inférieur en $C_1$-$C_5$ ramifié ou non.

16. Nouveaux medicaments, caractérisés en ce qu'ils contiennent au moins un composé selon l'une quelconque des revendications 1 à 15.

17. Médicament pour le traitement des troubles périphériques consécutifs au diabète, caractérisé en ce qu'il comprend au moins un composé de formule I ou un de ses sels non toxiques d'addition, tel que défini dans l'une quelconque des revendications 1 à 14.

**Revendications** pour l'état contractant: AT

1. Procédé de préparation de nouveaux composés répondant à la formule (I) suivante:

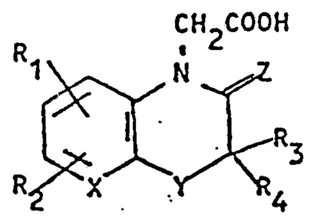

(I)

dans laquelle

Z représente l'atome de soufre, mais peut représenter également l'oxygène quand Y représente le soufre ou quand X = N et Y = NH;
Y représente l'atome d'oxygène ou l'atome de soufre ou un méthylène;
Y peut en outre représenter l'atome d'azote quand X est l'azote;
X représente CH ou l'atome d'azote;
$R_1$ et $R_2$ peuvent représenter l'hydrogène, un halogène, un groupement trifluorométhyle, méthoxy, thiométhyle, thiotrifluorométhyle, trifluorométhoxy;
$R_3$ et $R_4$ peuvent représenter l'hydrogène, un alkyle inférieur en $C_1$-$C_5$ ramifié ou non ramifié, un noyau phényle ou pyridyle; et leurs sels non toxiques d'addition,

caractérisé en ce que l'on réalise l'hydrolyse, en milieu basique ou acide, d'esters de formule:

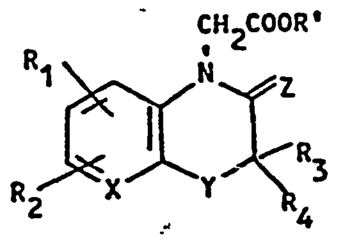

(II)

où $R_1$, $R_2$, $R_3$, $R_4$, X, Y et Z sont tels que ci-dessus définis et R' représente un radical alkyle inférieur en $C_1$-$C_5$ ramifié ou non.

2. Procédé selon la revendication 1, caractérisé en ce qu'on prépare les composés de formule (I) dans laquelle Z représente le soufre.

3. Procédé selon la revendication 1 ou 2, caractérisé en ce qu'on prépare les composés de formule (I) dans laquelle Y représente le soufre.

4. Procédé selon l'une quelconque des revendications 1 à 3, caractérisé en ce qu'on prépare les composés de formule (I) dans laquelle $R_1$ = H et $R_2$ représente le fluor.

5. Procédé selon l'une quelconque des revendications 1 à 4, caractérisé en ce qu'on prépare les composés de formule (I) dans laquelle $R_3$ = H et $R_4$ représente un méthyle ou un phényle.

6. Procédé selon la revendication 1, caractérisé en ce qu'on prépare un composé choisi parmi le groupe des composés répondant aux formules suivantes:

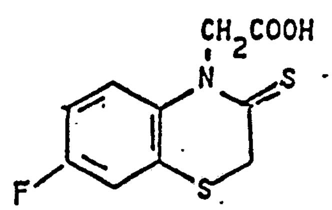

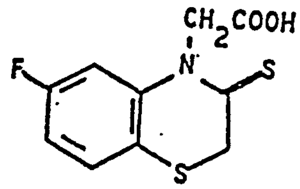

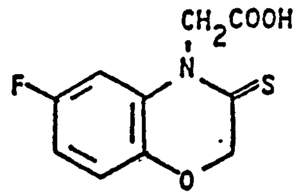

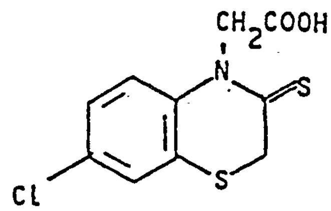

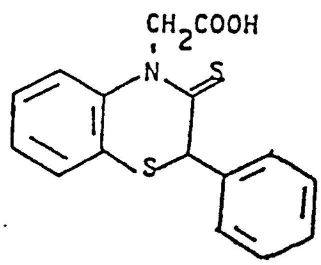

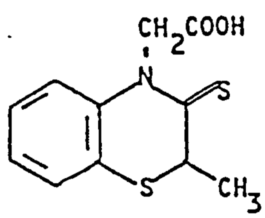

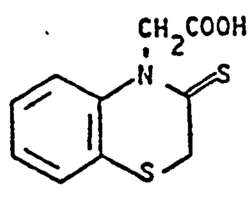

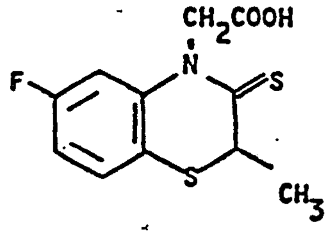

7. Procédé selon la revendication 1, caractérisé en ce qu'on prépare le composé pour lequel Z = S, Y = S, X = CH, $R_2 = R_3 = H$, $R_1$ = H ou halogène, et $R_4$ = H, méthyle ou phényle.

8. Procédé de préparation des composés de formule:

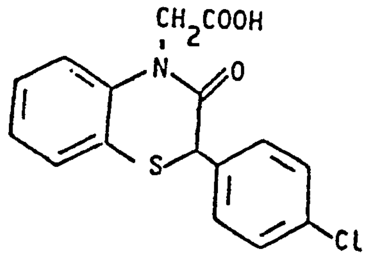

Code 5180-37

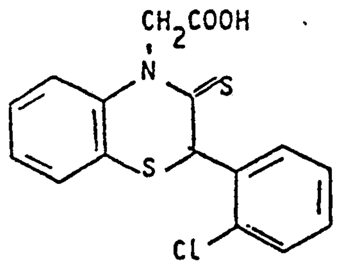

Code 5180-44

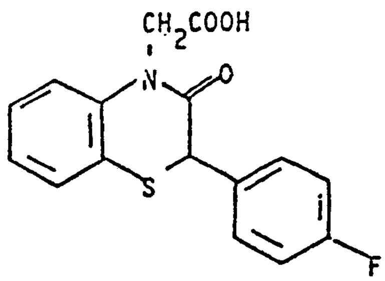

Code 5180-45

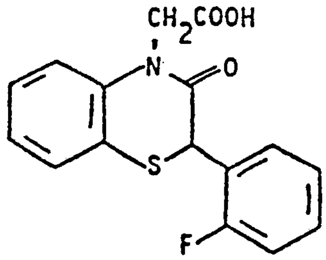

Code 5180-46

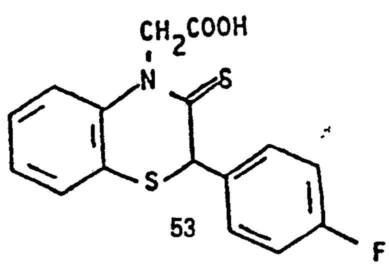

Code 5180-47

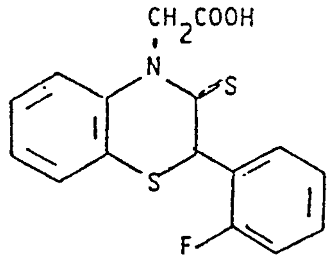

Code 5180-48

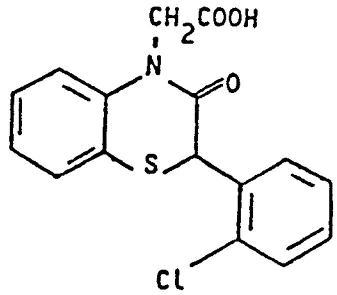

Code 5180-38

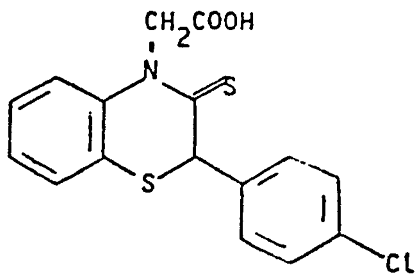

Code 5180-41

et leurs sels non toxiques d'addition, caractérisé en ce qu'on réalise l'hydrolyse en milieu basique ou acide, d'esters de formule:

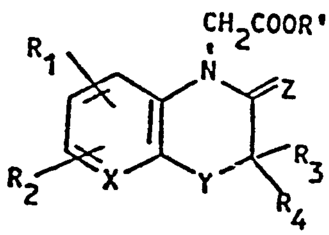

(II)

où $R_1$, $R_2$, $R_3$, $R_4$ X, Y et Z présentent les significations correspondantes et R' représente un alkyl inférieur en $C_1$-$C_5$ ramifié ou non.

9. Utilisation d'au moins un composé répondant à la formule (I) définie à l'une quelconque des revendications 1 à 8 incluant éventuellement des sels d'addition non toxiques, pour la préparation d'un médicament.

10. Procédé de préparation d'un médicament, caractérisé en ce qu'on mélange au moins un composé répondant à la formule (I), incluant éventuellement les sels d'addition non toxiques, défini à l'une quelconque des revendications 1 à 8 avec un excipient physiologiquement acceptable.

11. Procédé de préparation d'un médicament pour le traitement de troubles périphériques consécutifs au diabète caractérisé en ce qu'on mélange au moins un composé répondant à la formule (I), incluant éventuellement les sels d'addition non toxiques, défini à l'une quelconque des revendications 1 à 8 avec un excipient physiologiquement acceptable.

12. Procédé selon la revendication 10 ou 11, caractérisé en ce qu'on réalise le mélange sous forme de gélules ou comprimés renfermant 50 à 300 mg de composé de la formule (I) incluant éventuellement les sels d'addition non toxiques.

**Patentansprüche** für die Vertragsstaaten BE, CH, DE, FR, GB, IT, LI, LU, NL, SE:

1. Neue Verbindungen, dadurch gekennzeichnet, daß sie der Formel

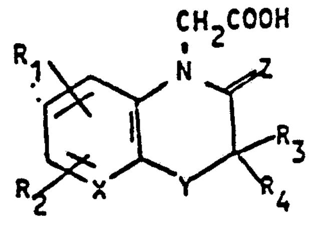

(I)

entsprechen, worin

- Z ein Schwefelatom darstellt, aber auch Sauerstoff bedeuten kann, wenn Y für Schwefel steht oder wenn X = N und Y = NH;
- Y ein Sauerstoffatom oder Schwefelatom oder Methylen darstellt; Y weiters für NH stehen kann, wenn X Stickstoff ist;
- X CH oder ein Stickstoffatom darstellt;
- $R_1$ und $R_2$ Wasserstoff, ein Halogen, eine Trifluormethyl-, Methoxy-, Thiomethyl-, Thiotrifluormethyl- oder Trifluormethoxygruppe darstellen können;
- $R_3$ und $R_4$ Wasserstoff, ein verzweigtes oder nicht verzweigtes $C_1$-$C_5$-Niedrigalkyl, einen Phenyl- oder Pyridylkern darstellen können,

und ihre nicht toxischen Additionssalze.

2. Neue Verbindungen nach Anspruch 1, dadurch gekennzeichnet, daß Z für Schwefel steht.

3. Neue Verbindungen nach Anspruch 1 oder 2, dadurch gekennzeichnet, daß Y für Schwefel steht.

4. Neue Verbindungen nach einem der Anspruche i bis 3, dadurch gekennzeichnet, daß $R_1$ = H und $R_2$ Fluor darstellt.

5. Neue Verbindungen nach einem der Ansprüche 1 bis 4, dadurch gekennzeichnet, daß $R_3$ = H und $R_4$ Methyl oder Phenyl darstellt.

6. Neue Verbindung nach Anspruch 1, dadurch gekennzeichnet, daß sie der folgenden Formel entspricht:

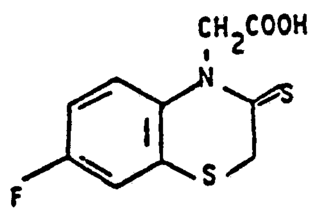

7. Neue Verbindung nach Anspruch 1, dadurch gekennzeichnet, daß sie der folgenden Formel entspricht:

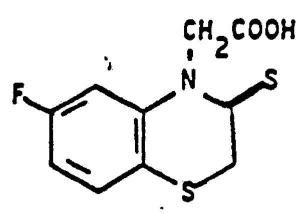

8. Neue Verbindung nach Anspruch 1, dadurch gekennzeichnet, daß sie der folgenden Formel entspricht:

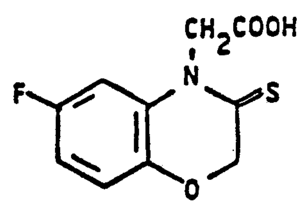

9. Neue Verbindung nach Anspruch 1, dadurch gekennzeichnet, daß sie der folgenden Formel entspricht:

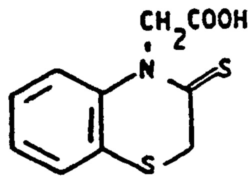

oder

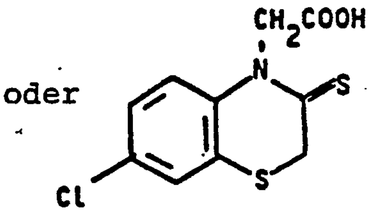

10. Neue Verbindung nach Anspruch 1, dadurch gekennzeichnet, daß sie der folgenden Formel entspricht:

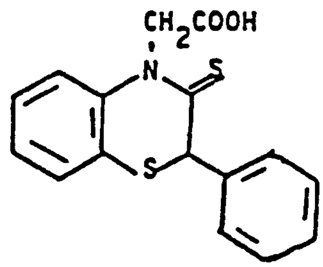

11. Neue Verbindung nach Anspruch 1, dadurch gekennzeichnet, daß sie der folgenden Formel entspricht:

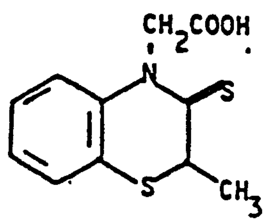

12. Neue Verbindungen, dadurch gekennzeichnet, daß sie ausgewählt sind aus den folgenden Verbindungen:

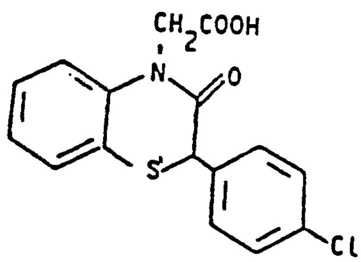

Code 5180-37

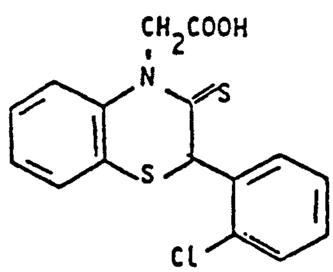

Code 5180-44

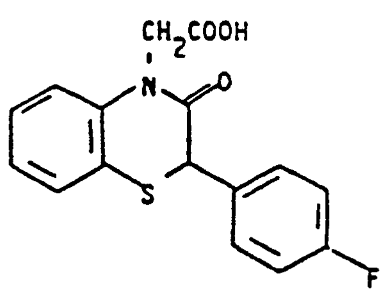

Code 5180-45

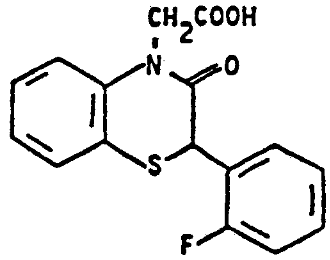

Code 5180-46

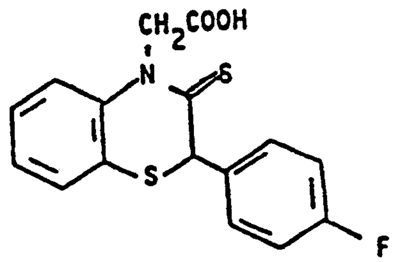

Code 5180-47

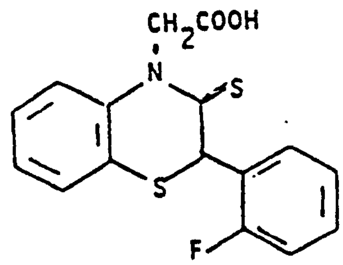

Code 5180-48

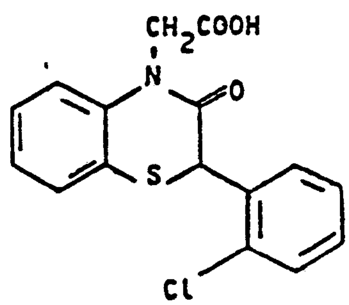

Code 5180-38

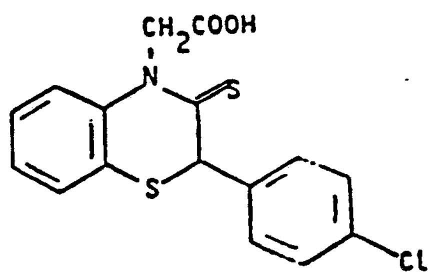

Code 5180-41

13. Neue Verbindung nach Anspruch 1, dadurch gekennzeichnet, daß Z = S, Y = S, X = CH, $R_2 = R_3 = H$, $R_1$ = H oder Halogen und $R_4$ = H, Methyl oder Phenyl.

14. Neue Verbindung nach Anspruch 1 oder 3, dadurch gekennzeichnet, daß sie der folgenden Formel entspricht:

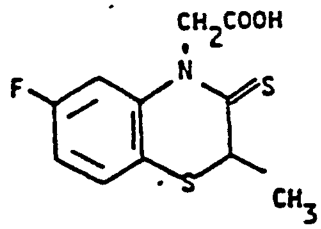

15. Verfahren zur Herstellung neuer Verbindungen nach einem der Ansprüche 1 bis 14, dadurch gekennzeichnet, daß man die Hydrolyse von Estern der Formel

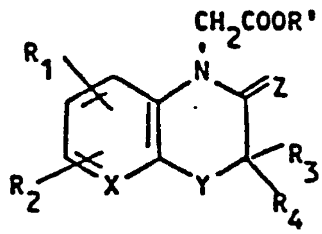

(II)

worin $R_1$, $R_2$, $R_3$, $R_4$, X, Y und Z wie in einem der Ansprüche 1 bis 14 definiert sind und R' für einen verzweigten oder nicht verzweigten $C_1$-$C_5$-Niedrigalkylrest steht, in basischem oder saurem Milieu durchführt.

16. Neue Medikamente, dadurch gekennzeichnet, daß sie mindestens eine Verbindung nach einem der Ansprüche 1 bis 15 enthalten.

17. Medikament zur Behandlung von peripheren Störungen infolge Diabetes, dadurch gekennzeichnet, daß es mindestens eine Verbindung der Formel I oder eines der nicht toxischen Additionssalze, wie in einem der Ansprüche 1 bis 14 definiert, enthält.

**Patentansprüche** für den Vertragsstaat AT:

1. Verfahren zur Herstellung neuer Verbindungen der folgenden Formel (I):

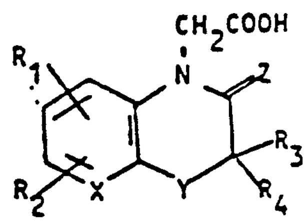

(I)

worin

| | |
|---|---|
| Z | ein Schwefelatom darstellt, aber auch Sauerstoff bedeuten kann, wenn Y für Schwefel steht oder wenn X = N und Y = NH; |
| Y | ein Sauerstoffatom oder Schwefelatom oder Methylen darstellt; |
| Y | weiters für NH stehen kann, wenn X Stickstoff ist; |
| X | CH oder ein Stickstoffatom darstellt; |
| $R_1$ und $R_2$ | Wasserstoff, ein Halogen, eine Trifluormethyl-, Methoxy-, Thiomethyl-, Thiotrifluormethyl- oder Trifluormethoxygruppe darstellen können; |
| $R_3$ und $R_4$ | Wasserstoff, ein verzweigtes oder nicht verzweigtes $C_1$-$C_5$-Niedrigalkyl, einen Phenyl- oder Pyridylkern darstellen können; |

und ihrer nicht toxischen Additionssalze, dadurch gekennzeichnet, daß man die Hydrolyse von Estern der Formel

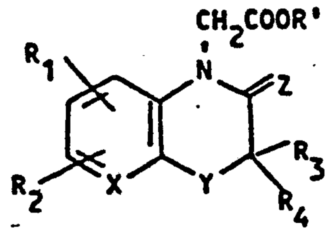

(II)

worin $R_1$, $R_2$, $R_3$, $R_4$, X, Y und Z wie oben definiert sind und R' fur einen verzweigten oder nicht verzweigten $C_1$-$C_5$-Niedrigalkylrest steht, in basischem oder saurem Milieu durchführt.

2. Verfahren nach Anspruch 1, dadurch gekennzeichnet, daß man Verbindungen der Formel (I) herstellt, worin Z für Schwefel steht.

3. Verfahren nach Anspruch 1 oder 2, dadurch gekennzeichnet, daß man Verbindungen der Formel (I) herstellt, worin Y fur Schwefel steht.

4. Verfahren nach einem der Ansprüche 1 bis 3, dadurch gekennzeichnet, daß man Verbindungen der Formel (I) herstellt, worin $R_1$ = H und $R_2$ Fluor darstellt.

5. Verfahren nach einem der Ansprüche 1 bis 4, dadurch gekennzeichnet, daß man Verbindungen der Formel (I) herstellt, worin $R_3$ = H und $R_4$ Methyl oder Phenyl darstellt.

6. Verfahren nach Anspruch 1, dadurch gekennzeichnet, daß man eine Verbindung, ausgewählt aus der Gruppe von Verbindungen der folgenden Formeln, herstellt:

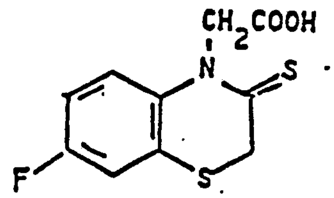

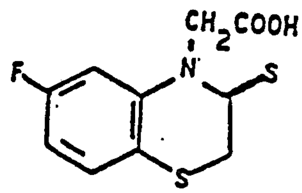

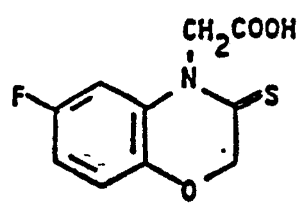

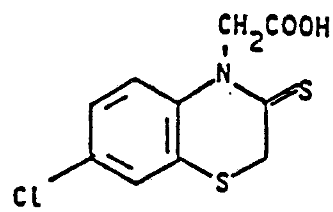

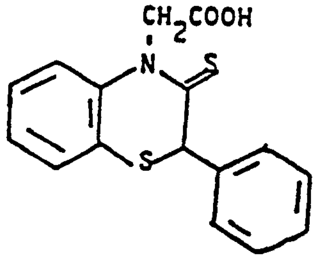

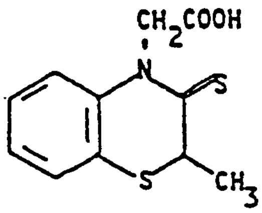

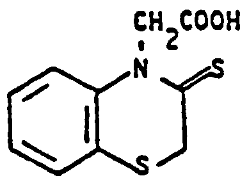

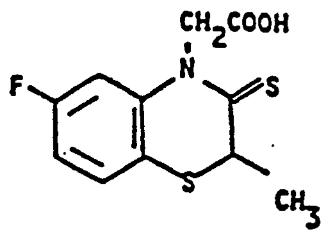

7. Verfahren nach Anspruch 1, dadurch gekennzeichnet, daß man die Verbindung herstellt, bei der Z = S, Y = S, X = CH, $R_2 = R_3 = H$, $R_1 = H$ oder Halogen und $R_4 = H$, Methyl oder Phenyl.

8. Verfahren zur Herstellung der Verbindungen der Formel:

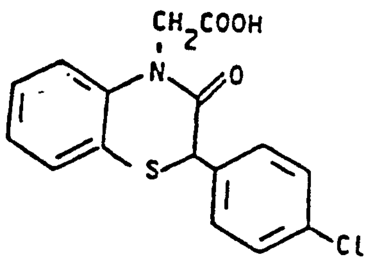

Code 5180-37

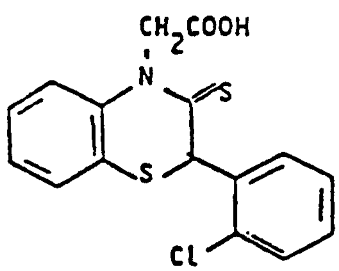

Code 5180-44

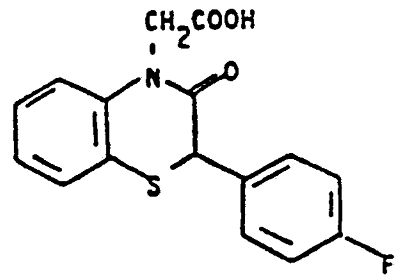

Code 5180-45

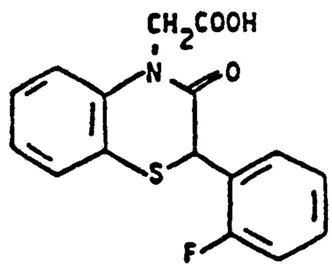

Code 5180-46

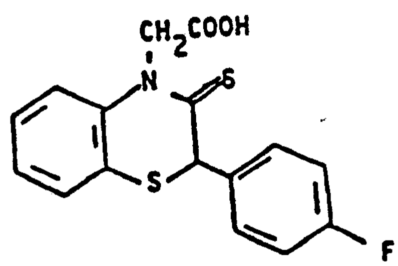

Code 5180-47

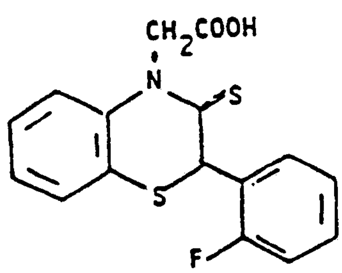

Code 5180-48

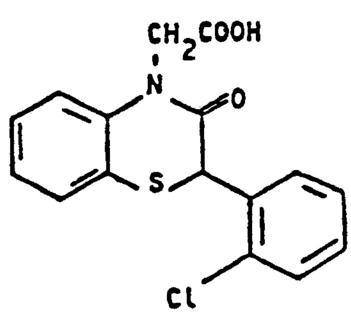

Code 5180-38

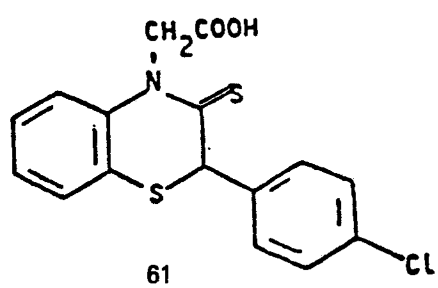

Code 5180-41

und ihrer nicht toxischen Additionssalze, dadurch gekennzeichnet, daß man die Hydrolyse von Estern der Formel

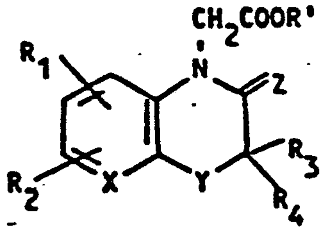

(II)

worin $R_1$, $R_2$, $R_3$, $R_4$, X, Y und Z die entsprechenden Bedeutungen haben und R' für einen verzweigten oder nicht verzweigten $C_1$-$C_5$-Niedrigalkylrest steht, in basischem oder saurem Milieu durchführt.

9. Verwendung mindestens einer Verbindung der Formel (I) nach einem der Ansprüche 1 bis 8, gegebenenfalls einschließlich der nicht toxischen Additionssalze zur Herstellung eines Medikaments.

10. Verfahren zur Herstellung eines Medikaments, dadurch gekennzeichnet, daß man mindestens eine Verbindung der Formel (I), gegebenenfalls einschließlich der nicht toxischen Additionssalze, nach einem der Ansprüche 1 bis 8 mit einem physiologisch akzeptablen Exzipienten mischt.

11. Verfahren zur Herstellung eines Medikaments zur Behandlung von peripheren Störungen infolge Diabetes, dadurch gekennzeichnet, daß man mindestens eine Verbindung der Formel (I), gegebenenfalls einschließlich der nicht toxischen Additionsalze, nach einem der Ansprüche 1 bis 8 mit einem physiologisch akzeptablen Exzipienten mischt.

12. Verfahren nach Anspruch 10 oder 11, dadurch gekennzeichnet, daß man die Mischung in Form von Kapseln oder Tabletten herstellt, die 50 bis 300 mg Verbindung der Formel (I), einschließlich der nicht toxischen Additionssalze, enthalten.

**Claims** for the contracting States: BE, CH, DE, FR, GB, IT, LI, LU, NL, SE.

1. New compounds characterized in that they are of formula:

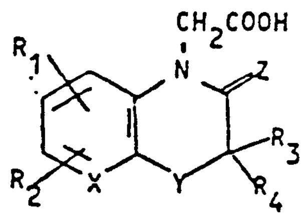

(I)

in which:

Z represents the sulfur atom, but can also represent oxygen when Y represents sulfur or when X = N and Y = NH;
Y represents the oxygen atom or the sulfur atom or a methylene;
Y can also represent NH when X is nitrogen;
X represents CH or the nitrogen atom;
$R_1$ and $R_2$ can represent hydrogen, a halogen or a trifluoromethyl, methoxy, thiomethyl, thiotrifluoromethyl or trifluoromethoxy group;
$R_3$ and $R_4$ can represent hydrogen, a lower alkyl in branched or unbranched $C_1$-$C_5$-chain, a phenyl or pyridyl nucleus and their non-toxic addition salts.

2. New compounds according to claim 1, characterized in that Z represents sulfur.

3. New compounds according to claim 1 or 2, characterized in that Y represents sulfur.

4. New compounds according to any one of claims 1 to 3, characterized in that $R_1$ = H and $R_2$ represents fluorine.

5. New compounds according to any one of claims 1 to 4, characterized in that $R_3$ = H and $R_4$ represents a methyl or phenyl group.

6. New compounds according to claim 1, characterized in that it is of formula:

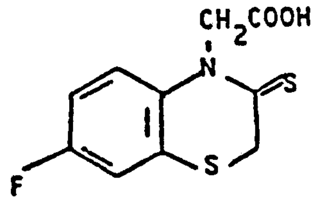

7. New compound according to claim 1, characterized in that it is of formula:

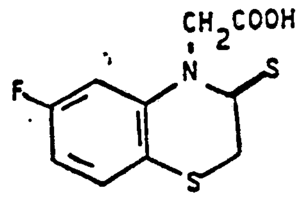

8. New compound according to claim 1, characterized in that it is of formula:

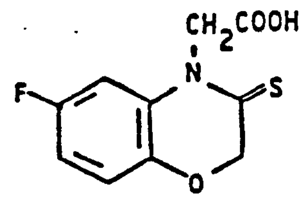

9. New compound according to claim 1, characterized in that it is of formula:

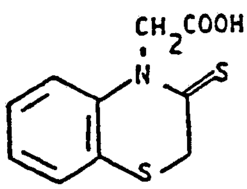

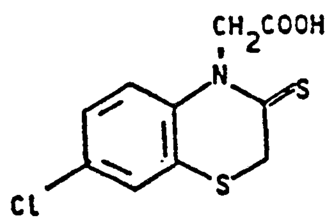

10. New compound according to claim 1, characterized in that it is of formula:

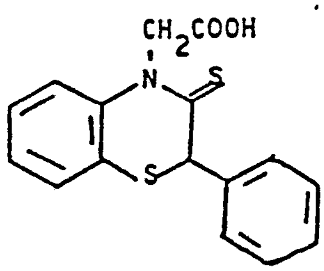

11. New compound according to claim 1, characterized in that it is of formula:

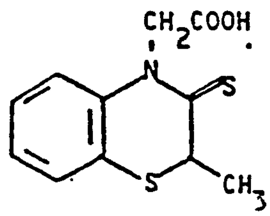

12. New compounds, characterized in that they are selected from the following compounds:

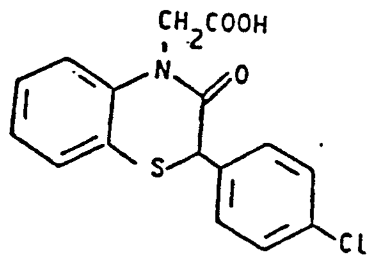

Code 5180-37

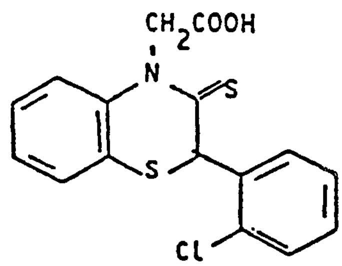

Code 5180-44

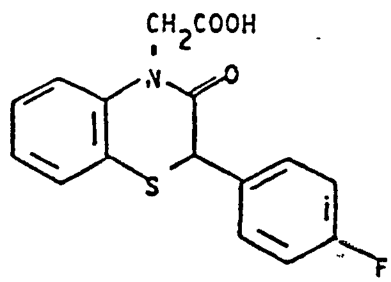

Code 5180-45

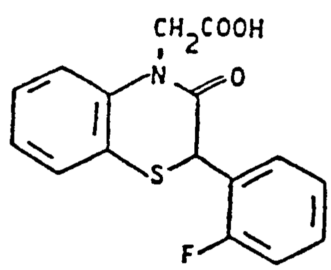

Code 5180-46

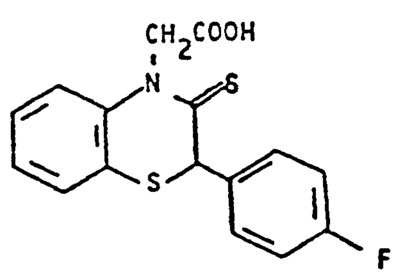

Code 5180-47

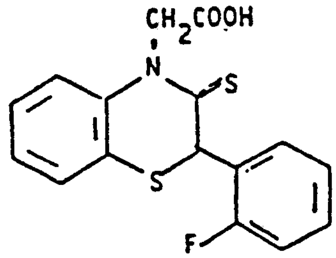

Code 5180-48

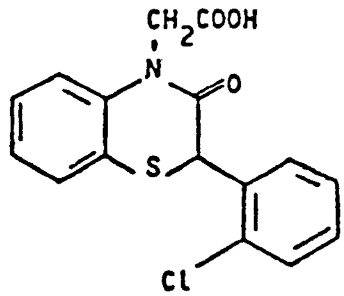

Code 5180-38

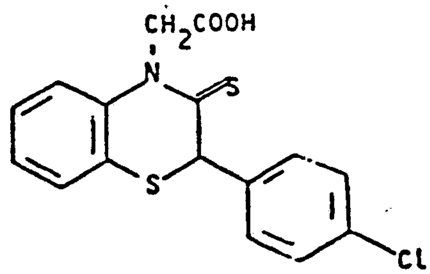

Code 5180-41

13. New compound according to claim 1, characterized in that Z = S, Y = S, X = CH, $R_2$ = $R_3$ = H, $R_1$ = H or halogen and $R_4$ = H, methyl or phenyl.

14. New compound according to claim 1 or 3, characterized in that it is of formula:

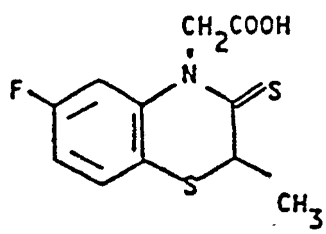

15. Process for the preparation of the new compounds according to any one of claims 1 to 14, characterized in that the hydrolysis is carried out in a basic or esters acid medium of formula:

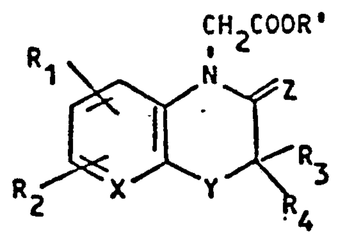

(II)

in which $R_1$, $R_2$, $R_3$, $R_4$, X, Y and Z are as defined in any one of claims 1 to 14 and R' is a branched or unbranched $C_1$-$C_5$ lower alkyl.

16. New drugs characterized in that they contain at least one compound according to any one of claims 1 to 15.

17. Drug for the treatment of diabetis-induced peripheral disorders, characterized in that it comprises at least a compound of formula I or one of its non-toxic addition salts, such as defined in any one of claims 1 to 14.

**Claims** for the contracting State: AT

1. Process for the preparation of new compounds of the formula (I):

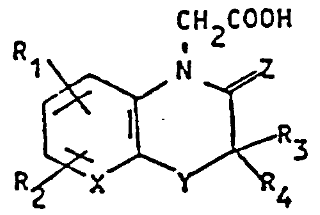

(I)

in which:

Z represents the sulfur atom but can also represent oxygen when Y represents sulfur or when X = N, and Y = NH;
Y represents the oxygen atom or the sulfur atom or a methylene;
Y can also represent the nitrogen atom when X is nitrogen;
X represents CH or the nitrogen atom;
$R_1$ and $R_2$ can represent hydrogen, a halogen or a trifluoromethyl, methoxy, thiomethyl, thiotrifluoromethyl, trifluoromethoxy groups;
$R_3$ and $R_4$ can represent hydrogen, a branched or unbranched $C_1$-$C_5$ lower alkyl or a phenyl or pyridyl nucleus, and their non-toxic addition salts, characterized in that the hydrolysis is carried out in a basic or acidic medium, of esters of the formula:

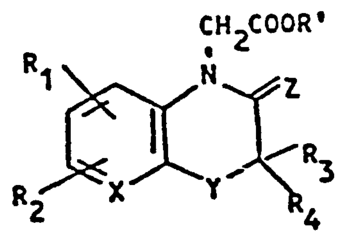

(II)

in which $R_1$, $R_2$, $R_3$, $R_4$, X, Y and Z are as defined above and R represents a branched or unbranched $C_1$-$C_5$ lower alkyl radical.

2. Process according to claim 1, characterized in that it comprises preparing the compounds of formula (I) in which Z represents sulfur.

3. The process according to claim 1 or 2, characterized in that it comprises preparing the compounds of formula (I) in which Y represents sulfur.

4. Process according to any one of claims 1 to 3, characterized in that it comprises preparing the compounds of formula (I) in which $R_1$ = H and $R_2$ represents fluorine.

5. Process according to any one of claims 1 to 4, characterized in that it comprises preparing the compounds of formula (I) in which $R_3$ = H and $R_4$ represents a methyl or phenyl group.

6. Process according to claim 1, characterized in that it comprises preparing a compound selected from the group of compounds having the following formulae:

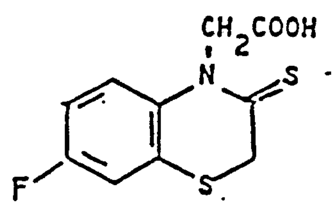

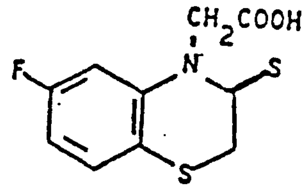

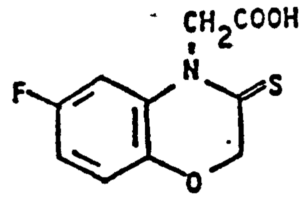

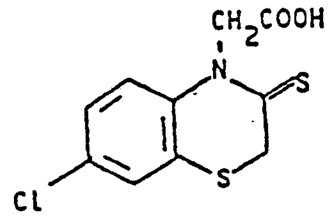

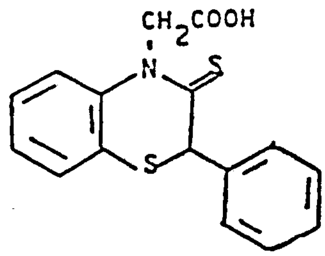

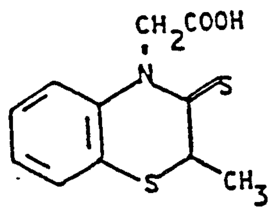

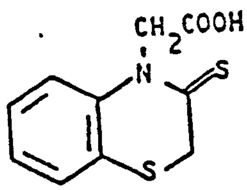

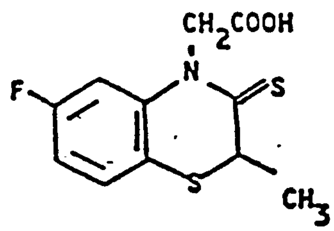

7. Process according to claim 1, characterized in that it comprises preparing the compound in which Z = S, Y = S, X = CH, $R_2 = R_3 = H$, $R_1 = H$ or halogen and $R_4 = H$, methyl or phenyl.

8. Process for the preparation of the compounds of formula:

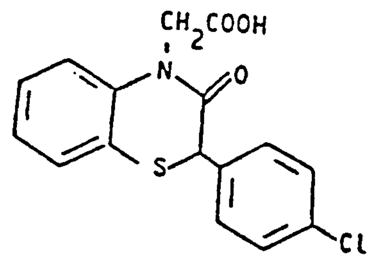

Code 5180-37

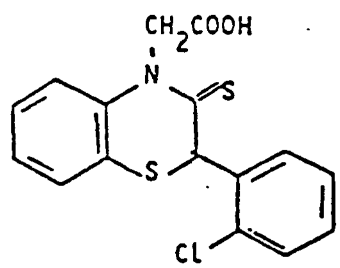

Code 5180-44

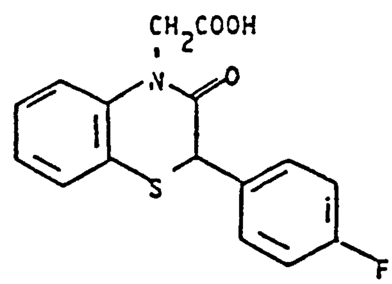

Code 5180-45

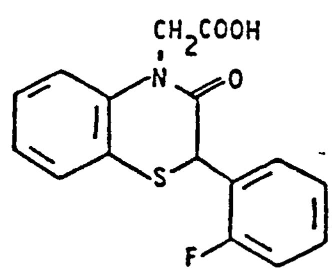

Code 5180-46

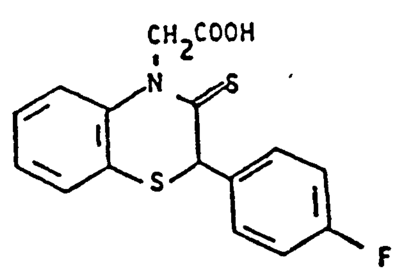

Code 5180-47

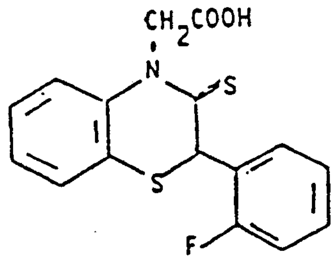

Code 5180-48

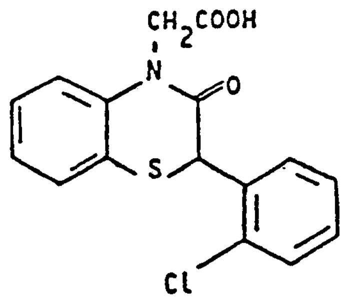

Code 5180-38

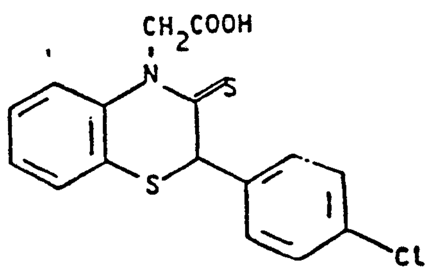

Code 5180-41

and their non-toxic addition salts, characterized in that it comprises hydrolyzing in basic or acid medium, esters of formula:

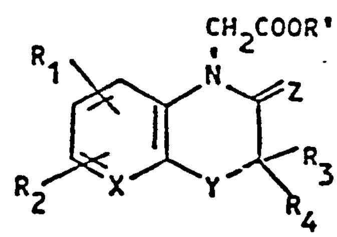

(II)

in which $R_1$, $R_2$, $R_3$, $R_4$, X, Y and Z have the corresponding meanings and R' is a branched or unbranched $C_1$-$C_5$ lower alkyl.

9. Utilization of at least one compound having the formula (I) defined in any one of claims 1 to 8 including optionally non-toxic addition salts, for preparing a drug.

10. Process for the preparation of a drug, characterized in that it comprises mixing at least one compound of formula (I), optionally including the non-toxic addition salts, defined in any one of claims 1 to 8, with a physiologically acceptable excipient.

11. Process for the preparation of a drug for the treatment of diabetis-induced peripheral disorders, characterized in that it comprises mixing at least one compound of formula (I), optionally including the non-toxic addition salts, defined in any one of claims 1 to 8, with a physiologically acceptable excipient.

12. Process according to claim 10 or 11, characterized in that the mixture is produced in the form of gelules or tablets containing 50 to 300 mg of compound of formula (I) including optionally the non-toxic addition salts.